# EUROPEAN PATENT APPLICATION

(11) **EP 3 002 010 A1**
(43) Date of publication of application: **06.04.2016**
(21) Application number: 14187205.1
(22) Date of filing: 30.09.2014
(51) Int. Cl.: A61K 31/7072, A61K 45/00, A61K 31/26, A61K 31/155, A61P 3/04, G01N 33/00, A61P 3/10, A61P 3/00

(54) **Food intake, body weight and glucose metabolism regulation by modulation of P2Y6 receptor signaling**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention is related to compound capable of regulating the activity of P2Y purinoceptor 6 signaling pathway, especially to compounds for inhibition of P2Y purinoceptor 6 polypeptide or inactivation, degradation, downregulation or intercalation of a nucleic acid encoding P2Y purinoceptor 6 or downregulation of P2Y purinoceptor 6 signaling pathway for the treatment of diseases related to energy balance as well as carbohydrate metabolism and homeostasis, preferably glucose metabolism and homeostasis. The present is also related to compounds for activation of P2Y purinoceptor 6 polypeptide or upregulation or modification for advanced transcriptional activity of a nucleic acid encoding P2Y purinoceptor 6, upregulation of P2Y purinoceptor 6 signaling pathway for gaining weight or for the treatment of diseases related to energy balance and carbohydrate metabolism and homeostasis. The invention is further related to methods of identifying said compounds suitable for the treatment of diseases related to energy balance and carbohydrate metabolism and homeostasis. The invention is further related to methods of treatment and diagnosis of diseases related to energy balance and carbohydrate metabolism and homeostasis and associated complications.

SEQUENCE LISTING

<110>  MPI GmbH

<120>  Food intake regulation by modulation of P2Y6 receptor signaling

<130>  GAR-P03707EP (II)

<160>  3

<170>  PatentIn version 3.5

<210>  1
<211>  2552
<212>  DNA
<213>  Homo sapiens

<220>
<221>  misc_feature
<223>  P2Y6 cDNA NCBI Accession: NM_001277204; Version:   NP_001264133.1

<400>  1
gtttgagtga gcaaacagag ggtgaatgaa gtgacttgcg ggcagctggg tagaggatga      60

gtcagcattg gtgtaaagga ctggagcttc agggttctcg ggattcgagt cctggccctg     120

ctgcttgcca gctctgtgct gtggcaagtt acttcctctc cccaggtctc tcggtttcct     180

catctgctgc ctctccagac ttctgccaga acattgcacg cgacagtttc aggcacagaa     240

ctgactggca gcaggggctg ctccacgagt gggaatttgc tccagcactt cacggactgc     300

aagcgaggca cttgctaact cttggataac aagacctctg ccagaagaac catggctttg     360

gaaggcggag ttcaggctga ggagatgggt gcggtcctca gtgagcccct gcctccctga     420

acataggaaa cccacctggg cagccatgga atgggacaat ggcacaggcc aggctctggg     480

cttgccaccc accacctgtg tctaccgcga gaacttcaag caactgctgc tgccacctgt     540

gtattcggcg gtgctggcgg ctggcctgcc gctgaacatc tgtgtcatta cccagatctg     600

cacgtcccgc cgggccctga cccgcacggc cgtgtacacc ctaaaccttg ctctggctga     660

cctgctatat gcctgctccc tgcccctgct catctacaac tatgcccaag gtgatcactg     720

gccctttggc gacttcgcct gccgcctggt ccgcttcctc ttctatgcca acctgcacgg     780

cagcatcctc ttcctcacct gcatcagctt ccagcgctac ctgggcatct gccacccgct     840

ggccccctgg cacaaacgtg ggggccgccg ggctgcctgg ctagtgtgtg tagccgtgtg     900

gctggccgtg acaacccagt gcctgcccac agccatcttc gctgccacag gcatccagcg     960

taaccgcact gtctgctatg acctcagccc gcctgccctg gccacccact atatgcccta    1020

tggcatggct ctcactgtca tcggcttcct gctgcccttt gctgccctgc tggcctgcta    1080

ctgtctcctg gcctgccgcc tgtgccgcca ggatggcccg gcagagcctg tggcccagga    1140

gcggcgtggc aaggcggccc gcatggccgt ggtggtggct gctgcctttg ccatcagctt    1200

cctgcctttt cacatcacca agacagccta cctggcagtg cgctcgacgc cgggcgtccc 1260

ctgcactgta ttggaggcct ttgcagcggc ctacaaaggc acgcggccgt ttgccagtgc 1320

caacagcgtg ctggacccca tcctcttcta cttcacccag aagaagttcc gccggcgacc 1380

acatgagctc ctacagaaac tcacagccaa atggcagagg cagggtcgct gagtcctcca 1440

ggtcctgggc agccttcata tttgccattg tgtccggggc accaggagcc ccaccaaccc 1500

caaaccatgc ggagaattag agttcagctc agctgggcat ggagttaaga tccctcacag 1560

gacccagaag ctcaccaaaa actatttctt cagccccttc tctggcccag accctgtggg 1620

catggagatg gacagacctg ggcctggctc ttgagaggtc ccagtcagcc atggagagct 1680

ggggaaacca cattaaggtg ctcacaaaaa tacagtgtga cgtgtactgt catcaagggg 1740

tatgctccat gctttgagtc accaatgaag cgggtgaggg aagatgagag ggggaggtga 1800

gagcttctgg gaaggggcat ttgagctggg ttttgaggga tgattatgag ctctctggag 1860

aggagtgata ttccatttat ttagaaagcc tttactgaca ccttgtgctc aggcctgtgt 1920

tggttctggg gccctagaag aaccagtcct agccctggtc catacgggct cccaactgct 1980

ggaagtacag actggcacag caacatcagg gttgtgacag agggaagcat ggctgggggg 2040

agggggtaca cagacagtgc ccatgaccca gtccaaggag tcaggaaaga gctctctgag 2100

gagggagcat ctgagccaga tgttgagggc tgagtgggaa cttggcaagc agaagtgggg 2160

agcactttaa tgcaacccag gtatgctcca tgcatatcca gctgggccag cctcgtgctg 2220

ggctctgccc tgggcagaca ggcagagggc cagagcagag gacacatggc cttgcgtgtg 2280

tgaaagctga gaaaatggga gctgtgcttc agctaccctc cagacaaggg caagagttag 2340

ccagatgctc caggcagtgg gaagccaatg gagggattaa gcggcggaag cttcttagaa 2400

gaggcagggg gcgaagtgca gtggctcatg cctgtaatct cagcaatttg ggaggccaag 2460

gaaggaggaa tgcttgagcc caagagtttt agaccagcct gggcaacaca gtaagaccct 2520

gtttctacaa aaaaatataa aaaatagcct gg 2552

<210> 2
<211> 328
<212> PRT
<213> Homo sapiens

<220>
<221> MISC_FEATURE
<223> P2Y6 protein NCBI Accession NP_001264133; Version:
NP_001264133.1

<400> 2

Met Glu Trp Asp Asn Gly Thr Gly Gln Ala Leu Gly Leu Pro Pro Thr
1               5                   10                  15

Thr Cys Val Tyr Arg Glu Asn Phe Lys Gln Leu Leu Leu Pro Pro Val
            20                  25                  30

Tyr Ser Ala Val Leu Ala Ala Gly Leu Pro Leu Asn Ile Cys Val Ile
        35                  40                  45

Thr Gln Ile Cys Thr Ser Arg Arg Ala Leu Thr Arg Thr Ala Val Tyr
    50                  55                  60

Thr Leu Asn Leu Ala Leu Ala Asp Leu Leu Tyr Ala Cys Ser Leu Pro
65                  70                  75                  80

Leu Leu Ile Tyr Asn Tyr Ala Gln Gly Asp His Trp Pro Phe Gly Asp
                85                  90                  95

Phe Ala Cys Arg Leu Val Arg Phe Leu Phe Tyr Ala Asn Leu His Gly
            100                 105                 110

Ser Ile Leu Phe Leu Thr Cys Ile Ser Phe Gln Arg Tyr Leu Gly Ile
        115                 120                 125

Cys His Pro Leu Ala Pro Trp His Lys Arg Gly Gly Arg Arg Ala Ala
    130                 135                 140

Trp Leu Val Cys Val Ala Val Trp Leu Ala Val Thr Thr Gln Cys Leu
145                 150                 155                 160

Pro Thr Ala Ile Phe Ala Ala Thr Gly Ile Gln Arg Asn Arg Thr Val
                165                 170                 175

Cys Tyr Asp Leu Ser Pro Pro Ala Leu Ala Thr His Tyr Met Pro Tyr
            180                 185                 190

Gly Met Ala Leu Thr Val Ile Gly Phe Leu Leu Pro Phe Ala Ala Leu
        195                 200                 205

Leu Ala Cys Tyr Cys Leu Leu Ala Cys Arg Leu Cys Arg Gln Asp Gly
    210                 215                 220

Pro Ala Glu Pro Val Ala Gln Glu Arg Arg Gly Lys Ala Ala Arg Met
225                 230                 235                 240

Ala Val Val Val Ala Ala Ala Phe Ala Ile Ser Phe Leu Pro Phe His
                245                 250                 255

Ile Thr Lys Thr Ala Tyr Leu Ala Val Arg Ser Thr Pro Gly Val Pro
            260                 265                 270

Cys Thr Val Leu Glu Ala Phe Ala Ala Ala Tyr Lys Gly Thr Arg Pro
        275                 280                 285

Phe Ala Ser Ala Asn Ser Val Leu Asp Pro Ile Leu Phe Tyr Phe Thr
    290                 295                 300

Gln Lys Lys Phe Arg Arg Arg Pro His Glu Leu Leu Gln Lys Leu Thr
305                 310                 315                 320

Ala Lys Trp Gln Arg Gln Gly Arg
                325

<210>  3
<211>  24759
<212>  DNA
<213>  Homo sapiens

<220>
<221>  misc_feature
<223>  P2RY6 gene NCBI Accession NC_000011.10; Chr 11:   72.98 - 73.01
       Mb

<400>  3
   agggaaggcc attagagtgg ggttgacgac agagaagaga ggtaaagcag ctcaagataa      60
   ggggccagag tggggagttc agggagctgc tgataagatt aaggcaggga aagtggggag     120
   cacgggagag gcgggatcct gagtaccctg ctttcctaac cacggtaatg gagagaggca     180
   cccccttccc ccactttcaa gcaagggagg tacgtcctta gcctggggtt ccgagcaggt     240
   atctcccagt aggtcccttc ctggccagcc gggctggggc aggggcggga caggggtagg     300
   gtggcgcggt ggctgggcgc aaaggtcccg cagtgggcca cgcaggcacc gggctgacct     360
   ggcaaaactt tggcgtctct gaaaacctct ggtaaccagc tcccttctag cgtgagggag     420
   ccgggaggcc tccttctggc ccggcagtga gagcgtcgcc gcccgaccct cccgttgcag     480
   caccggtaca gacacgctga ccccgcggcc ttgtcgctgg gcggcgtccc ggagcgggtg     540
   gcgcggtgtc tacccgggcg ggttgagggc ggtgccaggg tcagtcaaaa gtccgccccg     600
   ccccctgcct ggcccggctt cggggttggg gaacagcgca gggaggtggg tagccgggct     660
   cccaggcacg tgggtctctg cggctgcggc gggacccggg cactggcacc cgggagcggc     720
   ggcgacggca ccctgagagg agaagcgcag cgcagtggcg agaggtgggg tggggcgtct     780
   ggagggaaca aggcgctggg agaggcccgg ggaagcggcg ggccagactg gcgcaggtgc     840
   ggactgggcg cggggcaggg gagggcgctg tggagcggct gtcgccccgg ggcacagttg     900
   cgagccggcg tgtagcgcat aactggacgg cttgaacgcg cctgtgggtc cgtggttgaa     960
   ctttgtctcc acgggagcgt cggtctcggt gagtgtggac gggcgcccca gtagtgaggc    1020
   tgtgcgcgtc agcgtactgg ggaggtctgc gtgttagcgg gtgagtgtgt gcgcccctcg 1080
   gggcgactgg ccaagcctgc ggaacttgtc tgtgtgtgct ccgatgtgag cgtccccgtg 1140
   gcgtgaggcg gtggccggtg cccagagacg cgaacccctg gagaccccct accactgtcc 1200
   taaccccagg gtcactctgg gtggccacct accattggta atgttgaggg ggcagttgca 1260
   gggagtgggc gggggaggag ggagtggcca gaaaggacag ttaagcctct gtccccgggc 1320
   ttttaatctg gcaaatggtg cggtagaaag ggtcagtcag gagaaaggcg ataccccacc 1380
   ccccaacccg aaggctgcca gtggccctct tggggtcctc caggagcgca gcccaagggt 1440
   tggggactgc tggagaccag aattgccagc tcgggatttg tggggtccat ggctgctgtt 1500
   cctctcagca tggcatcccc agcttcccag gaccacagcc tgatctgcct ggtttgtttt 1560
   ggatctaagc tgaaataatt atgagttttg aggatctggg tggagaccca ggcaagttta 1620
   ttacctgtgg gtggaagtgg aaggaagaag attctctgag cagacaggtc cacaagagag 1680
   ctctggagac tcagagatct atgttccaat cctgtcttgg ccactgtgtg gcctgggcaa 1740
   gccgtccttt ctctgagcct cagtggcctc gtctgtaaga agggactaat gataaggaat 1800
   ttgagggctg ttgaggagct gatgagtgtg tcatgttcag caccagtgtc cccccatggg 1860
   caggctcaca gcttggggat gggaagtcag tccctggaag gagcctccca gaaccagaat 1920
   cccaactgtg cccctcccta gggcacaagc cttccacagg accaagcctg aaccctgtag 1980
   cctgggactt aaggtatttg ataactgggg cctgccagcc tttccagcca cctctcccgc 2040
   ctgactcttt accttgcagc agctgcctgg agtctctcat ttcttgcccc tggcctgttg 2100
   catattctac actccctctc tgataagccc tcctactctc accttcctca actggagaac 2160
   tacccagact tgggacccag ctcagatgtc ctctcctctg gaaagccttc cttgatccct 2220
   acagctgggt tggagcctct tctaggcccc tacaggcctg gggctgacag ccctggtccc 2280
   acctgaaccg tcatgatcta tgtctgtctc ctcctttcct cttcccaggt ctaggggctc 2340
   ctggagggca gaaactgggt cttattattt ctgtgttgcc agcaccctgc acagggctga 2400
   gctgaactga gagagcagca gtggcgccca tcccctgtct ctggcaccag cttctccagt 2460
   aaaggcccgg ctgcagggga gctccatgtc actacaaaga agggcaaacc agaatctggt 2520
   tatcaaagtg ggtggcacag tttgccagaa cagtgtgatc tgggacactg taggacctag 2580
   agtgcccagg ctaaagacct agagatttta tcctgaaggc aatagggagc catggaaatt 2640
   tatgagcaga ggtgtttcat ggtcagagcg gcatttcaga aagcgctctc tggctgcagg 2700
   atggagaatg gatcaagtat tgccaagtgc agggaggtca gggcatttgt gatgtcaaca 2760
   ctagaactgt cccaggagtt gtttgaaccc taattgagcc cttgcccctt ggccaaacac 2820
   ttttccctga gccttcagat gctcctgctg aggaatttgg ggacctgcaa ttgatctcat 2880
   cccagctctg ttcccagctg gttgaatgac cagttcctgc cctgccaggc tctaaaagga 2940
   ggccagaaca agtaagatcc tgcatgggag agtatgtgtg tggggaggct ctggcacaaa 3000
   aggcttgtcg ctctttctcc tcttctccct gccttgggct gtcttggagt ctggtcagag 3060
   gggttgggga tcccagtggg caaccctcag aaatgactac caagcttggg ctgtggagtg 3120
   aggctgtgac tcctctgttt ctgtctccct cctgccctgg atgcatgggg ctctaaggct 3180
   gtggatcccc caaggaccag ctaagaaggt gtgagggtat caccagggcc tcatgagtga 3240
   ccccatgaga gctttgactt aatggctata gtctttagag aggccattgt ccctctctag 3300
   gtgtcagttc tgccatttgt aaattgagaa tggtgatcct tgcttctttg agagcagagt 3360
   tgctacgagg attaagatag tgaccaggga agagctttat aagccttaaa ctgcagtgct 3420
   cagaggagga atgtgacact tgtagactta aatccatttt gaagactata agagaacatg 3480
   ggtgcaatag caagggattg tgcttgttat tgaaatttga ttactgaatg ccttcctccc 3540
   ccaccccctc tgcagagatg gcttcactag taacgattta caacaatcca ttttgtcttc 3600
   ctatagccct gtgagggagg aattgtatct ccattttgca gatcaggaaa tagaggcatg 3660
   gattgtatcc tgcccagggt caccaagctg aggtgaaacc tggggctcca gtccacctgc 3720
   cagcttagcc ctccccatct gagacagagc cctgttaggg ttaggcctgg tcctctggct 3780
   tccaggctga ggggagtttg aattctgccc tgggaacctg tgtttggggc tttcttgtgg 3840
   ccagattacc caactgctga taaggtaacg cctgcccaga cctgcccacg ggaccagaga 3900
   tgccttgttg atctggtgag gggagtgggg gttgcccctc atgccttcca cccaaaccgc 3960
   ctctccccaa actgctttct gtaggcctgc ttgctatacc ctctgggcca cacctcctct 4020
   tggttgtcct tgggtcctga ggcctggcct tcccttatta ctgggccctg ataggaggag 4080
   gctggctgtg tgggccactc tgacctctgt actcatccag cggccagttg caggactagg 4140
   ctaggccata cctgctttct ctctctgtcc ccatcagcca ccacaggttg ggctcagagc 4200
   agctgccagt gggtctttgg gaaatgagtg gccaggcggg agtaggcatg gagtagaagg 4260
   ggaggctttg ctccacggag tgccatagtt ggttctggag cagaggagtt cctccacccc 4320
   atcaccccat ctcctaggcc tgagtctttc ctggtccaag atgagtagcg ggagtttctg 4380
   gtaggagggt gagcagcctg ggccccgtgg aacaactcct tgaccctctg ccctggtacc 4440
   ttgtcactgt cattgtagct cctgaatgag aggcctgggt gctggagtga gggaaaggga 4500
   cctccctcag acttctgggg ctttatgtgg cctctccctc cccaggtctg aggagagcct 4560
   ctgggtgtcg acctggcttc ctggtgagtg cccagccacc cagccctggc ttgtaggagg 4620
   gggactcttg ccatggctgg gctgttgcag gaaatccagg gccaagggac ctgctcctct 4680
   catggcacca ccgtatgtgt cgtctcctcc ttggtctccc tccctccaga ctccctgtcc 4740
   catctacctt ccatgtggca ccatctttgt aaaggttgac tctgaccatg tccttcccct 4800
   cttaagcctt ccatggctcc tcagtgtcct tagaatagag gccaagcttt tcagcctggc 4860
   atttgagcct tttcaagtga attcctcagg ctcccacaca ttccatgcca cctcaccctc 4920
   cacagtagcc gtataagacc tcccagcctt tttccagcag gtcacacttt catccaccaa 4980
   gtctttgctc atgcctctcc tgccttcaga gccctcctcc ctctttgccc acctgacaaa 5040
   ctcctaatca tctaccagtt tccagctcag atgacccctc ctctgcagaa gcactccctg 5100
   accacctccc ccaggccagg taggagttgc tgtgtgctcc ataacccctg ggcttgcctt 5160
   ggttatggtt tattcaccca agtggatttg gtccatgtct gtgtcttttt cctcagcctg 5220
   actgggtact cctcaagggc ccagcagagt gcaatctgag tctgagttca caaggtccat 5280
   ctgaggccag gtgcaccaga gcattggcaa gtgtttgctg ggactttgtg gttcctggga 5340
   atgtgattta agatttcatt actctgaatc cagacccctg tggctctgag aatctcaaaa 5400
   ccaaaggcta tcaggagcaa cgagtaatag aattgaagaa acacagcctt cacacccgga 5460
   accagagcct cttggaatta gactgcgaga accagggaac tcagaatcct ggaggtacct 5520
   ggaggggcca tctcagaatc agaggctcat gtggttcctg gttgatgctt tcatccccct 5580
   ttccaccttg gattctccat ccaccttctt tcctcccaag ctggaccctt cctgctcctc 5640
   ctcaaatctt atcacagttc cccttccagg gaccttgcct gactgctctg accatgacct 5700
   ctgtccctct gctggggaac acctgggagg gcctccctac caagcagtgt catgggctcc 5760
   tgaatttcag tgtctccctg ttgtgacagg cagacacagc ctccacatta ggacagttca 5820
   tgatttgcta gcgccaaggt gcacccacct gcccccttgt cttattcctc atgagctgtc 5880
   ccatgcagca gtgattctcc ccatctgacc actgaagaac ccgaggctct gagaaggggg 5940
   gctgacttca ccaagactgc acagcacagg tggctccaag ttagagcctg aactcacggt 6000
   tcttgcctct tgaagctgga ggcatttggc tgtgataggt tggggtggtg tccaggacgt 6060
   tgtggttctg agcctgttta atttagtagc atgtctctgt agccctgttc tgggctatgc 6120
   tgggttccag gtgagatggg tggtttgggg tcctgctcta tgttgggggt gaggtggtca 6180
   aacccacact gacaataatt ggaacactag accttgagta aggcccttcc tctttagagg 6240
   tcttggcttt ctgatctgta agcctggttg atctgagact tacccactct gatgctgctc 6300
   tgagctcagc cttggtgagt ggcctccaca gccaggatcc taggggggtc ccaaagaggg 6360
   agagaacatc tgggaggggg tagagagccc aagggcctcc tgaagggggt gcctaacacc 6420
   acaacagcac cggctgatag catctgaggt taccccttag ccatttcctc agaccggccc 6480
   tggtgctccc ggtgcagaag catgtcaggc aaggcagggg gcctgctggc tttcagcccc 6540
   ttgaaggaca ttggggagga gccagcgtga cttctaagct cctcagcctg tgcactctag 6600
   ccttgccctc tccactcccc tcaagccacc aggatgatga gcagttcctc aaacgtgtca 6660
   tgtcatctct tgttcatcct cactctgctc ctcttctctc tccaaccacc tgactcctcg 6720
   tcttccaaaa tgcaggtcag gcgctctctc ctccaggaag ccttccctca tcacccctcc 6780
   aatcctgtta ggggcctcct tggcttcctc caggcctgat caccctgtgt tatcatcgcc 6840
   tgtttctgag tgtgcctccc ctgacgttga cagtgaattc cttgagatct gacctacctc 6900
   agagtcccca ggtgccctgc ccagcagtgg gcatagatgt ggcaaccttt gtgaagggtg 6960
   agcgagtgaa agagtatggt ggctatctgg cttctgctgg ggaatccctg gactgccttc 7020
   cccctgagga tggccttcag tgtgggagac ctgatctgtg gtttttctgg ttgtgacctt 7080
   cctgtatccc cttcccataa ttcctgcgaa gcttcctgcc cttcttgtct tcctgagata 7140
   accccaccct ccatgccttc tttacctgct gaaatattct ctcttactat ttcataaaat 7200
   aagcctaaat catctaacta gctcaacaac cttctgaggc aggtactgtt ctgatattga 7260
   gaggaaattg agatctgggt tttggagtta agagacttgt cttaccactg gaaagaggag 7320
   aactcggatt tcccacctgc ctagtgcctc tcttgccccc tcaatgaggt tctggcacta 7380
   ggtcctctgt tttccctcac agcacccaac tcctggcaga gataggcagc aggggtctcc 7440
   acagtggtga cagcacattc tcccctacca ggctagacag ggtctgccac cagccaggga 7500
   ggcacattcc agagccactc tagtggaggg gctggaatgt catccaacca ccctggaaat 7560
   tctgcagtgg cctgggagct gcccagctcc tcctcctgac tttggcctca ctagcctaga 7620
   accacaaaga gaaaggccca gactggggta ggggcagagc caggtcaggg ggttacaggt 7680
   ctgcatgcta ggcttggctc tgcccttggc tcactggtga ccttgggtga gaccttgagc 7740
   tacccctttc tggccttagt ttttctacct gtgagatggg tgcaataacc acccatctga 7800
   aaagagtaaa tcctgagccc tgccaattgg cattcctctg gcagagaact gagcagacag 7860
   gcagagagct gtggggccaa aaagggatgg tcaggcagct tcctgggaga gggccggggc 7920
   cttgcagggc aggaggtagt ggtcaggggc acagcctctg aaatcagaca gcccagagtg 7980
   gctatgccgt ttgctaattc aggattcaac gagaggaggt atatgaagcc acctaacaca 8040
   gaacttggca cagggacagc actaaactct tgttgtgata taggtgttag atgataaggg 8100
   aggccgtctc tgagcaatga tatgaccatg ggactgcttt tgttgtgcct gagacaggaa 8160
   gatgagggca gggacagaga ggatgggctt tctggaggga tggtgggtac cagatcagcc 8220
   agggcctgag tgccaggctg aggagctggg acagttgcct gagggcacta gggagccaca 8280
   aacatggctc aacaatgtat ggtaggattc gtgtttcagg aaactacatg actgcagtgt 8340
   gaaaggtgga cagacggggc aggggtggag gcacggaggc cagtgaggag gctgctgctg 8400
   tggactgtgg aagcacaggc ctcgggctgc agtgtgggga ccgcgtagtg catgtccgag 8460
   gaatttcact ggttggacta acagggcttg gtgagtggtg ggcttccagc agggtgggga 8520
   agagggaggt tgtccaggct gtatcagagg tttgaaacct ggaaggatgg tgggggccct 8580
   tctcagaaat gagaaccact tgtggaaagt gatattctcc attttagcct gttgagtcaa 8640
   ggcattttct aaggaggagg agacagggtg tgcacctggg tgcagcaggg tgaaaaggct 8700
   gaccatccct caccttgtca tagtctctag agtagatcag tggggagcta ccagcccacc 8760
   ggccttgtca atctgagacc accaggcaag gtgcactcag taagcagtgg cccagaggca 8820
   tttcccgtgt gcacccacgc cccccgtacc caagctattc ctgcagtgcc cccatctcct 8880
   ctcccacatc cccccagcct tgtactgcac ttccagcttg ggaacctggg ctcagctgtc 8940
   gcaggcccac tcctccatcc taggcaaatg aagatggaaa aagaaaaact caggaggtgc 9000
   atgttctatt tctcctgtga ggtacctgag gtgctggtgg agggtgggac ctgcccacac 9060
   agaaggtccc ctccaccagg caactgtccc ctccctgaaa ccctgtgctt ggtgtgcaca 9120
   aggggctcca aggtgagaca aaaaccacgg ccagaattcg ggagggctgg gctctagtct 9180
   tggctctgtc cctgacttac tgtgaggctt gggcctcagt ttcctcctct gtgaaacagg 9240
   gtttgacaca ggagatttct tttttttcta tatatatata ctttaagttc tgggttacat 9300
   gttcagaacg tacagttttg ttacacaggt atacacatgc cctggtggtt tgctgcaccc 9360
   atcaacccat cacctacatt aggtatttct cctaatgtta tccctcccct agccccacaa 9420
   ccccaacagg ccctggtgta tgatgttccc ctccctgtgt ccatgtgttc tcattgttca 9480
   actcccactt ataagtgaga acatgcggtg tttagttttc ttatcttgtg atagtttgct 9540
   gagaatgatg gtttccagct tcatccatgt ccctgcaaag ggcaggaact catccttttt 9600
   tatgactgca agtattccac gttgtatatg tggcacagga gatttctaaa tgccatttta 9660
   ggccctgatg gtctagactg tgtgtgtgtg tgtgtgtgca tccgtttgcg tccactgcat 9720
   gtacacctta gtgtgactct gcatgcctct gtgtatccgt gtgtcttggt gtgtctgtgc 9780
   cagtgtgtgt tcaggtgtgg catatgtgtg tttataacac ctaacgagga atgcttagag 9840
   gtaggagtgg ggaggagggg gtctctagat agaaacctag cttgggcaaa attgtattgg 9900
   cagaagggca aaagtgtgtt ggcagaagga caatagaatg cacactgggc aaagagggta 9960
   ttgacttttc ctgctatggg gccaaagcac agaaccagga ccgagggaag ggacactgga 10020
   ggcttggagg cttggctcag ctgtacagga catgggggag accttcctcc aggcagggcc 10080
   acccctctgt gaaaggcggc ctggggaagg gggttgtgga taacccattc ctggaatgca 10140
   tgagctgggg tttgggtgta cagaaggcac tcagaccagg acaaacacca ggccacctct 10200
   ctgcctcctt ctggttaagg ttaccacaaa ggaggtggcc ctggggggga ttgacctggg 10260
   taggtttcca tgaccctgag accgcacttt ctctacctcc ctctgtctgc aggagcccct 10320
   tgtggcagca gcactacctg cccagaaaaa tgctggaggc tgggcgtggc cccaggcctg 10380
   gggacctgtt tttcctgttt cccgcagagt tccctgcagc ccggtccagg tccaggcgtg 10440
   tgcattcatg agtgaggaac ccgtgcaggc gctgagcatc ctgacctgga gagcaggggc 10500
   tggtcaggta cgtggggtgg gggtgggggg gagcgggtac gctggccggg aggtgagggc 10560
   gggtgtgctg tcttgcctgt gtctgccctg aactggctta gatcaagcgg atctgaaggc 10620
   aggtgccctg ccctgctccc ccatcccagg ctgtgatgaa ttcaggcttt gaagccagat 10680
   agctctgggt gtcaagccta atttttccaa tgcccggcag tgtgaccttg tacaaatagc 10740
   ttaacctcta tggcaatatc taccaaggga tggaggtggg ggatgaagat gctgtgtgaa 10800
   agctgctcag cccaggtctg gcccttacaa agcagggtgg gtagagaatc ctgatcagac 10860
   ctattgaggt cctgagccag gcctcagaca gccaatccat ctccagagcg cccatgtcca 10920
   atcccctccc actggcactt ccctgacctg tccagagaaa ggcagagtct cctgggctcc 10980
   tgtggcagtt gaaggagaga cagtctagag acataagcag gttccaggtg gaccagaacc 11040
   cctagagggg ctgggttgtc ccctggttac ttagggtcag cagctaagaa acctttaagc 11100
   atttttgtcc ttgagaggcc ctcagaggag caagtaattt gtaagtaact tgatcaaggt 11160
   tacaccacgg gggatgggct gcaattccta ttcaggtcca tcccacagtg gaagtggctg 11220
   caggaggtag gtagtgaact ccctgtttat gggagtgtgc agaggctgca gagggcttgt 11280
   gcctgtgtgt gcctcctctg ggcccccata gcctcttgtg cttgcttcat tcattcattc 11340
   attcattcat tcattctctc attcctgtaa tacctcctgt ggtcaggcct cctgctggat 11400
   gatagggaca gagaaaagca tcagatggac ccagccctca aggagcacct gaaatgtgtt 11460
   ggggtagagg atggaaggac aaattaccaa ctggctgaga aacgtcctga ggggtgatgg 11520
   aagggagaac ttcagagatt agaagatcct ttgggaccta caatggggtg cagtgtgggt 11580
   gggttcgggg aaggctttcc agtgagaaag aacagcatga acacaggcag gggagcctgg 11640
   aagggtctta caaggctcac atctcccctc cagactggga gctccttggg gcagggccag 11700
   ccagaccagg gcgcagcggg gctggtgagc gttggatagt caggtggagc cgtctctagt 11760
   cccaggggca tcagggtcag gagaggcaga agggagactt aagggagggg gctggggact 11820
   tggctgtccc tctaaggcag gggctgtccc tctaaggcag ggctaggaaa gatatactca 11880
   caggttggtg agccagcctg tagaatgaga gggcgggagg ggtgagtctg gcccaggccc 11940
   acggcgatgg agtccccagc tggcctggga gggtgggggc cagggtgtct taaaacaggc 12000
   ggaaggggca gctctcttaa ttcaagggag acagggaagt ctccacccct catctcagtg 12060
   tcagagattc cactgttagg gattagcagg aaactcttca tgcagggtga tgagatgttt 12120
   atgttgactg tgaaggaaag tcaggcctcg gcacgggggc agaaacagag cagtggcgtg 12180
   aaggggagac tgggtgtgtg tgtctgcatc atggaggaag ccccctccct gtccaagtgg 12240
   ggcagaggac ctcccgggga tattttgggg ctgcagttct caaggctgtg ggatttgact 12300
   agtgtctgag acttgggagg gaaggaaatg cttgatgtca cctggcgttt tgtggcagag 12360
   ctagactcga acctcggctt actgataccc aggggaggct ctgtcctggg aaggaatgtg 12420
   gcctctccgt agctccttag tctcgcaagg gggcctctcc ttgctcctcc tgcagcttct 12480
   gtgagccagc gactctcagc tggccttcca aagctccaga cctgagggac ccagcctgtg 12540
   cccatcccca tacttgccat ggctgacact gcattcctcc tccccaccgt tcctccatca 12600
   gggaacagtc tccagagcct ccctcccaaa catctctccc ctcacagggc ccaaggcctt 12660
   cccctgcttg gctctcagtg tcactcccct ggctcaccac aatcacctcc acagcctcca 12720
   gtcccacctc cactgtggcc aaaggtttct ggaaaaggcc accgctagct gaaccttggc 12780
   actcataaca ccaccgtggg cctactgtgt gccaggctct actccagaca ttttatgcaa 12840
   atgactcgac cctcacagca gtcccatggt ggagattcta gtaattgtcc cattttacag 12900
   ctgaggaaac agaagcagtc tggctccagc gcccaggttc ttaatcactg ggttctctgc 12960
   actgagggat aaagagccgt gagtttaaca ggtggagacg agggctatga atgaactcca 13020
   ggtagaagaa gcagcagcaa ctaaggctgg gaggggagag tcttgggcac attcaagaaa 13080
   ctgcaggtca caggggttgg ggcgggtgag gttggcagag gctcagttat gaaaacctca 13140
   ggcaccagac ttgagggctg ggctttctcc tgaaacaggg taggagcggc aggaggattt 13200
   caagtagggg gatgctttct ttgagaaagg ccactctgga tttttgtggg tgggtgggtg 13260
   ggggtggcgc tggaggaggg aagccagtgc agtggttcca gcaacaagag gtagagcctg 13320
   gaacgaagtt ggcagcagga actgtcctga ccctgggtaa gacgggtctc agccacttga 13380
   cctgccagag ccccagcatc tacctgtctc ttcttaactt ggcaccacat aagcatctcc 13440
   cggcctcctc gccctggttt tgccctggct cctggggagc catctgtgtc catggcaggg 13500
   ggaacaaggg tgcaggcccc cttccttctg agtaaacatt tgctgacaat aggagaaaga 13560
   gaagatctgg gagtcttggt gaaagaaggg aaactggcca ggcgccatgg cccacacctg 13620
   taatcccagc actttaggag gccgaggtgg gtggattgct tgaggtcagg aattcgagac 13680
   caatgtgggc gacatggcaa aaccccatct ctactaaaaa atacaaaaaa aaaaaaaaaa 13740
   aagctaggtg aggtggcaca cacccgtaat cccagctact tgggaggctg agacatgaga 13800
   attgcttgaa cccaggagac agaggttgca ggaagctgag atcacgccac tgcattccag 13860
   tctgggtgat ggagtgagaa tctgtctcaa aaagaaaaaa aaaaaagaaa gaaaaaaaga 13920
   agggaaattg aaggatctct acacccccca gcacccctaa tctcagcctc ttgggccttg 13980
   aactgcctcc gggaagccag ggctggaaaa gcagctcagg gtctatggga gtagaggttg 14040
   caggccctgc agagtccccc agtcccttag aactccagga tctaaaataa agggaattat 14100
   tccaggcatg atggctcaca cctgtaatcc cagcactttg agagactaag gcgggcagat 14160
   cacttgaggt caggagttcg agaccagcct ggccaacatg gtgaaacccc atctctacta 14220
   aaaatataaa aattagccag gtgtggtagc atgtgcctgt aatcccaact actcaggagg 14280
   ctgagacagg agaatcgctt gaacctggga ggcagaggtt gcagtgagcc gagatcaggc 14340
   cattgtactc cagccttggc aacagaggtg gactccgtct aaaaattaaa aattaaaaat 14400
   aaacaaataa aataaaataa agggaattat acggccccag attctcagga tgaggaggac 14460
   tcactcttca tctgcccttt ccaccctccc agacacaccc ccacacaggc acaagccctt 14520
   ctgcagcctc tgctagagag ccacttgcat actcccataa acagggagtt cactaccacc 14580
   ctcctacagc cacttccagg ggctggacct ggacagatat tgcagcccat ccccagtggc 14640
   atgaccttga ccaaataact tacatgatct gtatcttagc ctcttgattg gagaactggg 14700
   gatggtgact tggcccccag aacacatcat atggataaag tgtagtaact tttttttttc 14760
   tgagacagag tcttgctgtg tcacccaggc tggagtacag tggcatgatt ttagctcact 14820
   gcaacctcca cctcctgggc ccaggtgatc ctcccacctc agacttccga gtagctggga 14880
   ctaccaccac accaggctaa ttttgtgtgt gtgtattttt agtagagaca gggtttctcc 14940
   atgttggcca ggttggtctt gaactcctgg cctcaagtga tccacccgcc tcagcctccc 15000
   aaagtggtgg gattacaggt gtgagccacc gcgcccggcc taagtgaata acttttagaa 15060
   agcatgctta gtagtagggt ttgaaaaatg ttagctcctc ctgccccttc acactgctag 15120
   aaagtgcttt tttgtgtttt tctcaaactt gcccacccag gacttctgct atgatttcca 15180
   acctgcctcc tgggacgtta ggcacgtctt tgtcttccag gccgggatgt ctgtcctcta 15240
   cctcccagct gcgagagagc tcttaattgg tcacctgggt aggggtaagg aggagaggga 15300
   ggggacaagc tgctccctgg gcactcactg tctgttggat ggtcagtccc catgggtgtg 15360
   ttatatgctc agtggggaca aggttggggg ggacaccatg atactgacga tatgcagaga 15420
   gaaactggat aggcatggaa gaagctgcat ttaccacaca aaagaagatc ccttggcccc 15480
   ctatccctat ctccaaccca gatttccctg gactcttcac gggggttact tcagggggat 15540
   tccctggtag cctcacaggc tcaggggcag gactgctgtt gatagatata gaaagtgcca 15600
   gaatagagga agatgtaaaa agtgaaggaa taactgaaga aggagaccct tggaaatact 15660
   tcctcaaata ccagaacact aggcatgggc ccagagcctc ccagagagtg ggaaaatgag 15720
   ctggagagaa ctctgtaatg cctgcagggg gctgttggat gcccactggc cagaaatgcc 15780
   tctgcaggca gtaggcattg ggtgtgaagc agggccagcc agcctcaaag gccccttccc 15840
   atgaactcaa aattacctgc tctggcaccc ccaggtcctc agattctaga gctcagaatc 15900
   ctagatctaa aactctgatc ctgaggttct agagaggggt aagaaaactt caagtagctg 15960
   ggactacggg tgtgtgccag catgcctggc taatttttaa aagaattttg tagagatggg 16020
   gtctcacttt gttgcctagg ctgggcttaa acacctggtt tctgccttgg cctcctgagt 16080
   ggctaggatt ataggcatga gccaccgtag ccagcttgag ctaacttttt ttaaaggtca 16140
   gatagtgaat attttagact tcacaggcca tatggtcttt atcacaacta ctcaactctt 16200
   tgtaaacaga tgggcaccgt tgtgttccaa caaagctgaa tttacaaaag cagactggat 16260
   ctggcctttg gactatagtt tgtctacccc tgttctcaag tttgacaatt gtgtgatcct 16320
   gatatttatt cattgatctc atgcattctc aaggttccag agcttggagg ttccaggtcc 16380
   agatccaagt cagatggcaa tgaaagccct ggtcaggtgg ctgtgtcagg tcccctaggg 16440
   gcgctccggc cacaaccctg atggccccac ccctcccttc cctgcagggc gatggcagca 16500
   gacctgggcc cctggaatga caccatcaat ggcacctggg atggggatga gctgggctac 16560
   aggtgccgct tcaacgagga cttcaagtac gtgctgctgc ctgtgtccta cggcgtggtg 16620
   tgcgtgcctg ggctgtgtct gaacgccgtg gcgctctaca tcttcttgtg ccgcctcaag 16680
   acctggaatg cgtccaccac atatatgttc cacctggctg tgtctgatgc actgtatgcg 16740
   gcctccctgc cgctgctggt ctattactac gcccgcggcg accactggcc cttcagcacg 16800
   gtgctctgca agctggtgcg cttcctcttc tacaccaacc tttactgcag catcctcttc 16860
   ctcacctgca tcagcgtgca ccggtgtctg ggcgtcttac gacctctgcg ctccctgcgc 16920
   tggggccggg cccgctacgc tcgccgggtg gccggggccg tgtgggtgtt ggtgctggcc 16980
   tgccaggccc ccgtgctcta ctttgtcacc accagcgcgc gcgggggccg cgtaacctgc 17040
   cacgacacct cggcacccga gctcttcagc cgcttcgtgg cctacagctc agtcatgctg 17100
   ggcctgctct tcgcggtgcc ctttgccgtc atccttgtct gttacgtgct catggctcgg 17160
   cgactgctaa agccagccta cgggacctcg ggcggcctgc ctagggccaa gcgcaagtcc 17220
   gtgcgcacca tcgccgtggt gctggctgtc ttcgccctct gcttcctgcc attccacgtc 17280
   acccgcaccc tctactactc cttccgctcg ctggacctca gctgccacac cctcaacgcc 17340
   atcaacatgg cctacaaggt tacccggccg ctggccagtg ctaacagttg ccttgacccc 17400
   gtgctctact tcctggctgg gcagaggctc gtacgctttg cccgagatgc caagccaccc 17460
   actggcccca gccctgccac cccggctcgc cgcaggctgg gcctgcgcag atccgacaga 17520
   actgacatgc agaggataga agatgtgttg ggcagcagtg aggactctag gcggacagag 17580
   tccacgccgg ctggtagcga gaacactaag gacattcggc tgtaggagca gaacacttca 17640
   gcctgtgcag gtttatattg ggaagctgta gaggaccagg acttgtgcag acgccacagt 17700
   ctccccagat atggaccatc agtgactcat gctggatgac cccatgctcc gtcatttgac 17760
   aggggctcag gatattcact ctgtggtcca gagtcaactg ttcccataac ccctagtcat 17820
   cgtttgtgtg tataagttgg gggaattaag tttcaagaaa ggcaagagct caaggtcaat 17880
   gacacccctg gcctgactcc catgcaagta gctggctgta ctgccaaggt acctaggttg 17940
   gagtccagcc taatcaagtc aaatggagaa acaggcccag agaggaaggt ggcttaccaa 18000
   gatcacatac cagagtctgg agctgagcta cctggggtgg gggccaagtc acaggttggc 18060
   cagaaaaccc tggtaagtaa tgagggctga gtttgcacag tggtctggaa tggactgggt 18120
   gccacggtgg acttagctct gaggagtacc cccagcccaa gagatgaaca tctggggact 18180
   aatatcatag acccatctgg aggctcccat gggctaggag ccagtgtgag gctgtaactt 18240
   atactaaagg ttgtgttgcc tgctgagctg tgccctattg tgtggtcggg ggatgaggat 18300
   atggcaggga agctttcacc agccacacaa gggtcctttc tccaatccgt tcccttctgc 18360
   cacctgcctt ctcactagct gtctcaggag tagtctcata tcagggatcc tctctccagg 18420
   ccccaggtca tcccccactg taaagccagt tggcttctgt gcctgactct gtgctgagca 18480
   cagagaaaag tcaggtgcag tcccagtcct tgagatttcc cagtttaggt gatggccagt 18540
   catgtgctgg taaatgttaa gccatttaac tggagctccg atttaactgg gaaccccttc 18600
   tttgtagaac tgcccatttc catggtgcaa aaactcttat ggccaaattc aaactataaa 18660
   catgatgtca acttccttgt aaaattcctg aaaatttaac aattggttct tgcaagcagg 18720
   tacaagtcac tctagcacac cactggataa tgccgagtgg ctggggctgt gagccagggg 18780
   gtcagggaag ggttcctgga gtgggagact cctgagctgt agcttctgag aaaagcagct 18840
   caccggaaga acatccacac acaggatgca tcccaggcaa agacatgggg caagaggcca 18900
   gggtgtgcat gagccataag caggctggac agctgactcc aggctcaagg cagggttggg 18960
   gctgggtcac aaggaggcca agttagggct ccctccttgc cctgaccctg aggcttcctt 19020
   tgcactgggg gtaaatatga aagagattca ctcctcctcc tgtccatcgt ctgccttctg 19080
   ggaaaatccc agaatggcag ggtggtgctc aggctgggtc aggacttagt atgggggaga 19140
   tgggtctcac ctatgatagg ttctgagtct agccaggacc actctgggct gacgtgtggc 19200
   gctcagctgg acagggcccc gccctagcct ttggaaaggg acagggcaaa gctgacaggc 19260
   ctcactcttg atctcaagga cagggactcc ctcctctccc tctgggagag ccctcgccct 19320
   gtggcccact ctgcctgccc cctctgacct ctccaccctt cccactctgc cttgtgaaag 19380
   gcaggacatc cccaaagctt gctgtatttg gagcctgact ccacagcgcc ctcatgtgac 19440
   agagacccat cacagaccat gacccaaatg attactctgt actgtgccag gtgggtgacc 19500
   tgcccagaat agtgtgagct attcacctct caccttctag gttctatact tctgttaatg 19560
   tagccgatgt ccttctgagt tttttttttc tttttgagac gaagtcttgt tctgtcaccc 19620
   aggctggaag tgcagtgctg cggtcttggc tcactgcaac tctctgcctc ccgggttcaa 19680
   gcaattctcc tgcctcagtt tcccaagtag ctgggattac aggtatgcgc caccacacct 19740
   ggctaatttt tgtattttta atagagacag ggtttcgcca tgttggccag gctggtctca 19800
   aactcctgac ctcaagtgat ccacctgcct cagcctccca aagtgctggg attacaggta 19860
   tgagccaccg cacccggccc ctcttgagct ttcttaatga ttgcctgtta caaaccaccc 19920
   ttgagccttt cccacaggcc cgagccactc cacaaggaca caggaggggc tcgttcacca 19980
   tctaatcctg tggcaggcac aggtgggggt ggatactgga gaccctgggt gaaccagggg 20040
   cacctctgga acccagttgg actgcctggc cccttggtat gcacgtccca ccttgctgct 20100
   cagtgcccag gccacttgcc ttgtgggtga agttcctgcc ctgatgcctc atgctatttt 20160
   cagcccaagt aagaccataa ttttccactc ctggccccag accattgtcc agcagtgacg 20220
   tgaggggatc tgcctgccct caaaatatct cctattaccc tccaatattc cctgccccct 20280
   tcacacctcc ctaacctgac atcacttcct gggctgttga ctcatgaagg ccactgtgaa 20340
   agggcacgtg agttgcccat ccactgagca gaccccaggg attatccagc ccagctgcag 20400
   actcaaggcc agagatgggc agggctgagc ccaaggtcat tcagcaggtc agcgtggcgc 20460
   ctggatgtaa gcaagtgcct tggtgacgat cctgctgtca ctcacactgc ccagctcttc 20520
   ctgcccaggg tgactgggat ggagccatag acctgaggcc agggacagga cagggatggg 20580
   aggaaaggga gggtagagag ttgagtgtca gggagtaaat gtctgctgat gtgcctgcag 20640
   cagctgttct caatgaactg catgacatca gggcagggtc cctggcacac aggggtctct 20700
   gccctgggtg gcaggtgtcc cagggtccag gagggatgga ggaaccccag ggtacattcc 20760
   cactttcacc ccctgaggtt agaagaggcc ctaagcaaca tgttatccac acatggttgc 20820
   tccaggtcaa gtcaccaaag ctacctctct gtcacccttg tcatggcaga tttgggctgc 20880
   tctgtttaga atcaggcaca tgagggaggg aagtcgtgta ttgacccatg tcccacgcac 20940
   cctctcatct atgttatatg gtggatgctt cacagaagcc ctgggaagta gatgctatga 21000
   tcctatttag cagatgagga aacaggagaa gggcgtgaga agtgaggact tgttccaggt 21060
   tatccaggct gtcagggtga gtcagagtga gtccaggttc acctgatggc cagaatcatg 21120
   ccctttatag tgccccacga cactcacagc cctgcagggc agaagggtct ataagtggcc 21180
   cctcgcatga ttcaagatga cagaggcagt gtagcaagga tgtgagaaca cagatgctcc 21240
   acccagacca cctcagtgtc aatattgtcc ccactttcct ggctggggtc tcttgggcaa 21300
   gttacctaat cttgcagcac ctcagttttc ccatttctaa agtgtgctgc taccttccat 21360
   gcagggcgtt gaggggcttc cctacgttat tacgcacaaa gtcctcctca cagtgcctgg 21420
   tgtgtggtca gcatttatat gcctggcacc aggccggggg ctatggaggc aaatcagctg 21480
   ggccctgccc tcacagagtt gacatcactt agtacgtaac cacattggga tacatgtttt 21540
   gaaggagagg acagtgtgtc tggccagtga gcaacagtgg gccatacgtc tccagggaac 21600
   ccccgtgggg tgtgggagga gggaagcagg tctgtcctgg gggaatgtgt gggaccacct 21660
   ctgccctgct tggcagtagg tggatttcag gaccatgggc ctgtgctgtt tacagaacct 21720
   tgtccaggtg cccagctgtg ggagggcagc atggagagga ggaagagggg gcccctagta 21780
   gcaagcttca gaaacttgcc acttcaggct agagagctcg caccctccca cggcagcagg 21840
   gtagccattt ctccctgact ggggtgtcca ccatggtgct ctgcagccac ctctcacttc 21900
   attaagagtc cacagatcta ggagcagagg actggtctgg agctgggcaa gggcaggcag 21960
   caaatgggga gtttttgctg tgtgacctga ggtcacttgc ctgccttctc tggactgcac 22020
   tgtagggcct ggagacctgt tcccctgttc caatttcccc acctcagtga aggcacaacc 22080
   aacagctgct ccccgggcat ttccaagacc ctccaggccc ccagctctga ggactagggt 22140
   ggaggcagtg tttctcccca gcatcaagtg accagagaag tgaagtgacc ccactgccgc 22200
   cacacagagc cacacagtgc gatgtctgga gctcctgcct cctgcaaggt ggagggtggg 22260
   gcttggccag gagtgaccag actacagagt gaggggtgtg cttgtggggg tgaagggttg 22320
   gggtgagaac tgagcccgag tcgaactcat cctcctctgc cctgatgcac tgtgcaactt 22380
   gggggagtca ctgtccctct ctgggcctcg ctttccccat cttcgatagg acttggctct 22440
   gcctggccac tcctcctcca atgggggaat tcctgggctt gcctctgtct tggctccagc 22500
   tcaggtgtca gtctcagccc tgtcccagcc aggaacaatg ggccaggcag ggccagtcac 22560
   cccctgtccc tgggggctcc acccacggcc ctggactatt ttcagccctt gcagatggag 22620
   tcagaagagg attttccact cccagtacaa tgactgccct ggaccattgt ccagcagtga 22680
   cgcgggggac ctgcctgccc caaaatatct cctgccccta gtttccaagc ctctttgccc 22740
   tgctcctcac ctcctggccc tgctccttac ctccccaccc tgatgtcatt tcctggggct 22800
   cttaactcct gaaggccatt gtgaaacatc acgtgagact cctgggagac acaaccagtt 22860
   ggaatgagtt ttccctcctg gcagggaccc cagggattgt ttggctgctt agtgggactg 22920
   gtggcccaga gagggctgtg ccttacccag tcacacagca gtcagtgtga agcttaggtg 22980
   taagcccagg gctctttggc tgctcccact gcctcagcca acttcattag ctccagtctg 23040
   tggctcctgg actggagcta cagtggtgag agctcaccac tgagcttcct gggatggcca 23100
   cctgggatag cagcccaagt ggtgtgggtg gagaggtgca gctcatgctc tgaccttgga 23160
   gtgagcccag tgcaggggcc tgaccttaaa ccctttctcc ccactgagtc ccatttttac 23220
   caaatgtggc tgttaggaac tgaatgtttg tgtcccccgc cccacaaatc catatgtcaa 23280
   aatcccaact gccaaggtga tggtattaga aggtggggcc tttgggaagt aaccaggtcc 23340
   taagggtgca gccctcatga atgggtttag tgcccttaca gaatggaccc cagagagctc 23400
   ctcaccctct ttccaccatg tgaggatata atgagaagtt ggcagtctgc aacctggaag 23460
   aggtccctca ccagatccca accatgctgg caccccgatc ttagacttcc agtcttcaga 23520
   atggtgagaa ataaatgtct cttgtttcta atccacccag tcggtggcat tttgttatag 23580
   cagcccacgc tgactgtgac tgaactcagg gtgacagagc agcagctctg ggcaaagggg 23640
   gagggtgggg gccacagggc agctgatgca ggagcagggc tctggcacag ttcagccatc 23700
   accaattctg ggactgcaaa ttcagtttct tcatctggaa aattgaactg gtaataccaa 23760
   tacctctttt cccggtttac tagacagaga caatgcagtg aggcacaaac atgcaactgt 23820
   ttgattttcc tcccacccat gctccctgct cagtgacgtg tgattttcct ttcccatcca 23880
   taaactgcgc atttggcaaa ctccaaactt ttgagtcact tttgggtaac aaagtcaggt 23940
   ttaagatttc catcgcctgc ttaattgaat gctcacctgt cctcttcccc catgctgtga 24000
   ggaccttcat cactcctctt ctgggtccct ctctcctcta ggtgttgggg cagggtgggg 24060
   aggaaaggga ccaatgtgtc tttcctggtg atgctacagc cctctcttgc tgaccttgct 24120
   tcctgggcca tcagtgcccc ctgcacagaa ctctccaaac atcagtccac acctttgtaa 24180
   aaacagtctc tccgctaagc tatcccaaaa tgacctgatt agagtgtgca acgtctttcc 24240
   tgctgggact ctgactgata ccctggtctc cctgagtgat tttcctgcac accctgctgg 24300
   ggatgtggaa gcctgtcccc ctcccacccc acaccccaca cttggctgag atctcacagt 24360
   ggcaaagatg tctttgaatc catggtgccc agggttcagc gtgtagagtg aggtggggtg 24420
   tgtagagagc tggggcaagg aaccctgaga agatgtctta ccccagttgt ttccaggttt 24480
   ctttagaaag cgggctattt taccccatgt gtcctcagat ttatgtcctg gaaccaaatt 24540
   ccaaatcagc agccacatga aaagtcccct cctctctggc gtggaattaa actgttagac 24600
   ctgcctttgt gaccaaagca attgtaaaac gtgggggagg aaattaaaag cctgccacct 24660
   tagtccacac agtgtgtgct gtctctgtgt gtgtgcacgt gtgtttatgt gtgtgttgaa 24720
   tgatcacatg ttccagggag gatggtgagg ggcgagggg 24759

## Description

The present invention is related to compounds capable of regulating the activity of P2Y purinoceptor 6 signaling pathway, especially to compounds for inhibition of P2Y purinoceptor 6 polypeptide or inactivation, degradation, downregulation or intercalation of a nucleic acid encoding P2Y purinoceptor 6 or downregulation of P2Y purinoceptor 6 signaling pathway for the treatment of diseases related to energy balance as well as carbohydrate metabolism and homeostasis, preferably glucose metabolism and homeostasis, such as obesity, type 2 diabetes mellitus (T2D), and related complications selected from cardiovascular diseases, hepatic steatosis and lipid disorders. The present is also related to compounds for activation of P2Y purinoceptor 6 polypeptide or upregulation or modification for advanced transcriptional activity of a nucleic acid encoding P2Y purinoceptor 6, upregulation of P2Y purinoceptor 6 signaling pathway for gaining or maintaining weight, such as anorexia nervosa and cancer cachexia.

The invention is further related to methods of identifying said compounds suitable for the treatment of diseases related to energy balance and carbohydrate metabolism and homeostasis. The invention is further related to methods of treatment and diagnosis of diseases related to energy balance and carbohydrate metabolism and homeostasis, such as obesity, type 2 diabetes mellitus (T2D), and related complications selected from cardiovascular diseases, hepatic steatosis and lipid disorders.

Diabetes as a leading cause of death in developed countries is a metabolic condition characterized by high blood sugar levels. There are two main types of diabetes: type 1, resulting from insufficient insulin production of the pancreatic beta cells, which requires the person to inject insulin; and type 2, resulting from insensitivity of peripheral tissues to insulin (such skeletal muscle, liver or adipose tissue), insulin release alterations, and relative insulin deficiency.

Type 1 diabetes is a genetic or autoimmune disease; the only effective therapy to date is the supply of exogenous insulin. This therapy does not cure type 1 diabetes; the person needs continuous supply of insulin.

The decreased insulin sensitivity of peripheral tissues in T2D that accounts for 90% of all cases of the disease is initially compensated by an increased release of insulin by the beta cells of the pancreas. At a certain stage of the disease, the pancreas cannot maintain the increases release of insulin anymore. T2D is often acquired and accompanied by obesity; it can be treated in first hand by reducing weight, diet and exercise. Patients diagnosed with T2D often require pharmaceutical medications to control their symptoms.

In the midst of the present escalating prevalence of obesity and T2D, there is an urgent need for unraveling the exact mechanisms underlying the control of body weight and glucose homeostasis. Researches led during the past decades pinpointed the critical importance of the central nervous system (CNS), and more particularly the hypothalamus, in homeostatic processes governing energy balance and glycemic control. Particular attention has been paid to the pivotal role of the arcuate nucleus of the hypothalamus (ARH), which contains two main antagonistic neuronal populations: the orexigenic neurons co-expressing neuropeptide Y (NPY) and agouti-related peptide (AgRP) and the anorexigenic neurons that synthesize proopiomelanocortin (POMC). These two populations of neurons are well described for being direct targets of the adipoctyte-derived hormone leptin and the pancreatic hormone insulin, both of which are anorexigenic and secreted proportionally to body fat mass. The discovery that obesity and T2D are associated with the onset of neuronal leptin and insulin resistance complicates the understanding of these ARH neuronal circuitries and largely limits pharmaceutical interventions targeting these hormonal pathways.

The inventors identified that a G-protein coupled receptor, the purinergic receptor 6 (synonymously called P2Y purinoceptor 6 or P2Y₆), is highly expressed in the hypothalamus. It was found that P2Y₆ displays a specific regional pattern of expression and is particularly highly expressed in the ARH. P2Y₆ is part of the purinergic receptor family that contains some of the most abundant receptors in living organisms and is highly conserved throughout evolution. The metabotropic P2Y₆ receptors are G protein-coupled receptors and are mainly responsive to uridine diphosphate (UDP), for which they are the sole receptors.

The inventors further explored P2Y₆ and its ligands, one of those is UDP, and found that P2Y₆/UDP pathway is involved in metabolic regulation and therefore can control the onset and the progression of diseases related to energy balance and carbohydrate metabolism and homeostasis such as obesity and diabetes.

The objective of the present invention is to provide targets for the treatment of diseases related to energy balance and carbohydrate metabolism and homeostasis and related complications. This goal is achieved by the claimed compounds, which regulate the activity of P2Y purinoceptor 6 signaling pathway. Further advantageous embodiments, aspects and details of the invention are evident from the depending claims, the description, the examples and the figures.

### Short description

The invention refers particularly to a compound for use in therapy of diseases related to energy balance as well as carbohydrate metabolism and homeostasis and complications associated, wherein the compound regulates hypothalamic level of UDP. That compound may be a regulator of uridine transport to the CNS (transport over the blood brain barrier) or a regulator of the synthesis, respectively the enzymes important for the synthesis of UDP in the CNS. In the CNS only a low level of *de novo* pyrimidine synthesis has been reported. Most of the pyrimidine (including uridine) content in the CNS is supplied by the uptake of pyrimidine nucleosides. Uridine-phosphorylating enzymes (uridine kinase and UMP kinase) are of low affinity. Consequently, providing the CNS with uridine increases formation of UDP and thereby UDP levels. Thus, regulation of uridine transport to the CNS is a promising target for influencing UDP levels in the CNS and consequently regulating P2Y purinoceptor 6 signaling pathway. Receptors transporting uridine and being responsible for the transport of uridine into the brain are concentrative nucleoside transporters (CNT 1 - 3; Na+-nucleoside transporter) and equilibrative nucleoside transporters (ENT 1 - 4).

The invention refers further to a method for screening for a compound for treatment of diseases related to energy balance and carbohydrate metabolism and homeostasis, wherein the method comprises providing a test compound for contacting at least one P2Y₆ polypeptide or nucleic acid coding for P2Y₆, detecting the binding of said test compound to the P2Y₆ polypeptide or nucleic acid coding for P2Y₆ polypeptide, and determining the activity of the P2Y₆ polypeptide in the presence of said test compound.

The invention refers also to a compound that regulates the activity of P2Y purinoceptor 6 signaling pathway. In a preferred embodiment of the invention said compound is suitable for inhibition of P2Y purinoceptor 6 polypeptide or for inactivation, degradation, downregulation or intercalation of a nucleic acid encoding P2Y purinoceptor 6 for the treatment of a disease related to energy balance and carbohydrate metabolism and homeostasis.

Said compound can be chosen from the group comprising a small molecule, an RNA molecule, a siRNA molecule, a miRNA molecule, or a precursor thereof, an antisense oligonucleotide, an aptamer, a polypeptide, an antibody, or a ribozyme, wherein RNA, peptides, small molecules and aptamers are preferred compounds.

The invention refers further to a pharmaceutical composition comprising a compound for the treatment of diseases related to energy balance and carbohydrate metabolism and homeostasis, more preferred of obesity or diabetes, more preferred of diabetes mellitus type 2, and related complications selected from cardiovascular diseases, hepatic steatosis and lipid disorders.

The invention further provides a method for treatment of diseases related to energy balance and carbohydrate metabolism and homeostasis comprising administering a subject in need thereof a therapeutically effective amount of at least one compound for inhibition of P2Y purinoceptor 6 polypeptide or for inactivation, degradation, downregulation or intercalation of a nucleic acid encoding P2Y purinoceptor 6.

In another preferred embodiment of the invention said compound is suitable for activation of P2Y purinoceptor 6 polypeptide or for activation, or upregulation of a nucleic acid encoding P2Y purinoceptor 6 for the treatment of a disease related to energy balance and carbohydrate metabolism and homeostasis, in particular for use in a therapy gaining or maintaining weight which is desirable for the treatment of diseases such as anorexia nervosa or cancer cachexia.

The invention further provides a method for treatment of cancer cachexia, underweight, treatment of underweight by newborns, treatment of underweight by people in extreme need of care comprising administering a subject in need thereof a therapeutically effective amount of at least one compound for activation of P2Y purinoceptor 6 polypeptide or for activation, upregulation of a nucleic acid encoding P2Y purinoceptor 6.

### Description

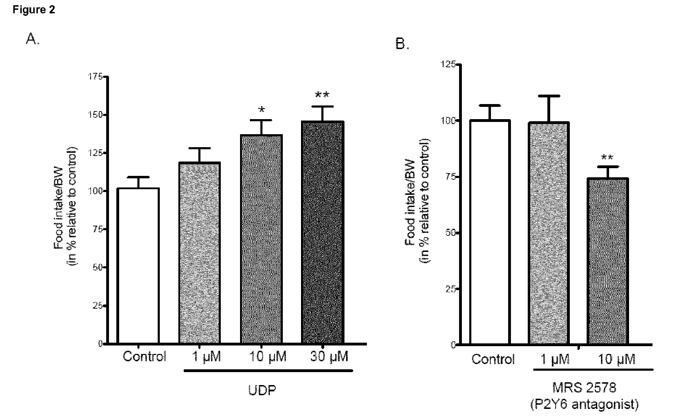

It was surprisingly found that UDP plays a direct role on the central regulation of feeding behavior, shown as effect of intracerebroventricular administration (ICV) of UDP on spontaneous food intake in wild type mice fed a normal chow diet. ICV administration of UDP increases food intake in a dose-dependent manner (Figure 2A). ICV administration of a non-competitive selective antagonist of P2Y₆ decreases food intake (Figure 2B). This set of data shows that central UDP and P2Y₆ modulate feeding behavior and that inhibition of P2Y₆ is able to decrease food intake.

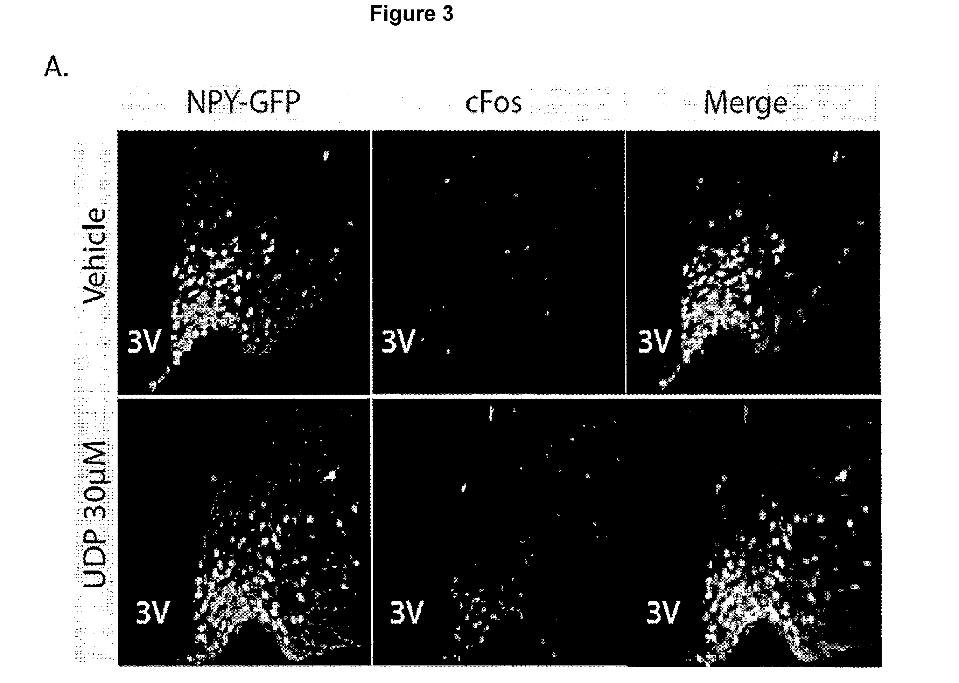

Furthermore, the inventors could show that UDP directly activates orexigenic NPY/AgRP neurons via activation of P2Y₆, which is in perfect accordance with its ability to promote feeding (Figure 3A-E). Surprisingly, the inventors discovered that UDP/P2Y₆ are new players in the central modulation of food intake and pinpointed a novel pathway controlling NPY/AgRP neurons. Data presented here reveals that UDP/P2Y₆ signaling in the brain is strongly involved in feeding regulation and that abnormal UDP levels might contribute to the development of obesity and T2D. In this line, the discovery that pharmacological inhibition of P2Y₆ decreases food intake suggests putative value in cases of hyperphagic obesity. AgRP-neurons are also known for acting as a key player in the brain-liver axis as they directly controls hepatic gluconeogenesis. Based on the UDP-induced activation of NPY/AgRP neurons and the well described role of this neurons in the maintenance of euglycemia, antagonism of P2Y₆ will not only be beneficial to regulate feeding behavior but also to control alteration of glucose homeostasis, such as occur in T2D.

According to the invention, regulation of hypothalamic UDP level and P2Y purinoceptor 6 receptor pathway are thus preferred targets for a therapy of obesity and diabetes, preferably diabetes type 2, and associated diseases. Therefore the present invention refers to a compound that regulates the activity of P2Y purinoceptor 6 signaling pathway.

The present invention refers particularly to a compound for
a) inhibition of P2Y purinoceptor 6 polypeptide or
b) inactivation, degradation, downregulation or intercalation of a nucleic acid encoding P2Y purinoceptor 6,
c) downregulation of P2Y purinoceptor 6 signaling pathway
d) inhibition of uridine transport across the blood brain barrier,
e) inhibition of UDP synthesis in the CNS,
f) acceleration or increase of UDP degradation
for use in the treatment of diseases related to energy balance and carbohydrate metabolism and homeostasis, preferably including obesity and type 2 diabetes and associated diseases.

Thus, in a preferred embodiment of the invention, the action of the receptor P2Y6 is blocked by an inhibitor or even more preferred by an antagonist.

Hence, one preferred embodiment of the present invention refers to an inhibitor of P2Y purinoceptor 6 polypeptide for the treatment of a disease related to energy balance as well carbohydrate homeostasis and metabolism, notably obesity and type 2 diabetes and associated complications.

It is sufficient to block activity of P2Y₆. However, inhibition of uridine transport over the blood brain barrier, the UDP synthesis or activation of UDP degradation in the CNS may exert a similar therapeutic effect. Receptor inhibitors are, in general, molecules, which bind to receptors and decrease their downstream signaling. The binding of an inhibitor can stop a natural ligand, such as UDP, from entering its binding site, for example by binding the receptor and causing a conformational change of the receptor in a way that the ligand cannot bind. Alternatively, the inhibitor may compete with the natural ligand for the binding site, or hinder the receptor from its reaction to ligand binding, e.g. an enzymatic reaction or a conformational change. Inhibitor binding can be reversible or irreversible. Typical inhibitors of a receptor are antagonist.

A compound according to the present invention can be a molecule which interacts directly or indirectly with the target polypeptide, such as P2Y₆, wherein, as a consequence of the interaction, the activity, e.g. the downstream signaling pathway is inhibited, blocked or has decreased activity. An inhibition of P2Y₆ may be reversible or irreversible or may be competitive or allosteric.

A compound according to the invention may interact with the P2Y₆ polypeptide, e.g. by binding the P2Y₆ polypeptide in a manner leading to conformational changes, masking, binding and/or degradation of the target. Compared to an activity level observed in untreated cells or organism, a decreased signaling activity means a change or decrease in the activity of so called second messenger downstream of P2Y₆ and/or downstream effectors by at least factor 1.5, preferably by factor 2, more preferably by factor 5.

If the target structure is a nucleic acid such as DNA or RNA, such as P2Y₆ encoding DNA or mRNA, a compound of the invention can be a molecule interacting directly or indirectly, e.g. intercalating with the target nucleic acid, wherein as a consequence of the interaction, the expression, i.e. transcription and/or translation, of said target nucleic acid is inhibited or downregulated, preferably inhibited. A regulator directed against a target nucleic acid may also be a molecule, which enhances the cleavage or degradation of this nucleic acid.

The term "P2Y₆ polypeptide" refers to the P2Y purinoceptor 6, while the term "P2Y₆ nucleic acid" refers to a nucleic acid such as DNA or mRNA encoding the P2Y purinoceptor 6.

A compound or regulator according to the invention may be selected from:
(i) nucleic acids, in particular small interfering RNA (siRNA), micro RNA (miRNA) or a precursor thereof, oligonucleotide aptamers, anti-sense oligonucleotides, or ribozymes;
(ii) peptidic compounds, in particular antibodies or antibody fragments or peptidic aptamers;
(iii) small organic non-peptidic molecules, i.e. molecules having a low molecular weight;
   and
(iv) combinations thereof.

Such compounds or regulators may have the ability to specifically regulate at least one target as described herein, e.g. due to binding to the at least one target.

The term compound as it appears herein refers inter alia to a molecule that is able to change or regulate or modulate the activity of the P2Y₆ polypeptide. This change may be an increase or a decrease in signaling activity, binding characteristics, functional, or any other biological property of the polypeptide. In order to reduce food intake, body weight and/or improve glucose homeostasis, inhibition of the P2Y purinoceptor 6 is advantageous.

In another preferred embodiment, the action of the P2Y purinoceptor 6 is impeded by interference to its nucleic acid, which can be both DNA and RNA, by inactivation, degradation, downregulation, or intercalation. Inactivation of a nucleic acid can happen for instance by methylation of nucleotides, insertion, deletion, nucleotide exchange, cross linkage, or strand break/damage. Downregulation of DNA or RNA is referred to as diminished expression of these nucleic acids and can happen by binding of repressors, which are usually polypeptides, but can also happen by chemical or structural changes or modifications of the nucleic acids. Intercalation is the reversible inclusion of a molecule between two other molecules. In nucleic acids, intercalation occurs when ligands of an appropriate size and chemical nature fit themselves in between base pairs.

According to the invention, compounds for the inhibition of P2Y purinoceptor 6 polypeptide can be molecules like small molecules, RNA or DNA molecules, siRNA or precursor thereof, miRNA or precursors thereof, ribozymes, DNA or RNA antisense oligonucleotides, aptamers, antibodies or fragments thereof, peptides, polypeptides, cyclopeptides.

The inventive compounds are also referred to as regulators. They regulate the expression and/or activity of the P2Y purinoceptor 6 polypeptide and can be identified using one or more assays, alone or in combination. Test compounds used in the screening are not particularly limited. They can be either artificial or natural.

The term small molecule refers to low molecular weight organic compounds being by definition not a polymer. In the field of pharmacology, it is usually restricted to a molecule that also binds with high affinity to a biopolymer such as proteins, nucleic acids, or polysaccharides. The upper molecular weight limit for a small molecule is approximately 200 Da, which allows for the possibility to rapidly diffuse across cell membranes. Small molecules are broadly used as enzyme inhibitors or receptor blockers, thus they are preferred regulators for the inhibition of P2Y purinoceptor 6 polypeptide in the present invention. Preferred small molecules according to the invention are selected from general formula (I) 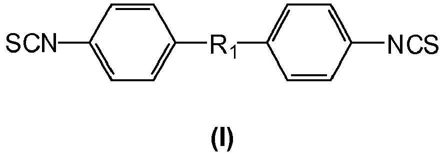 wherein
R₁ is selected from: trans-CH=CH-, -CH₂-CH₂-, -NHCSNH(CH₂)₂NHCSNH-, -NHCSNH(CH₂)₃NHCSNH-, -NHCSNH(CH₂)₄NHCSNH-
or said compound is selected from general formula **(II)** 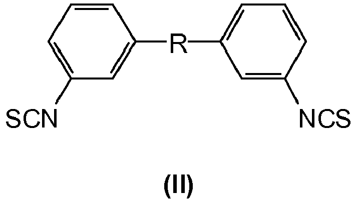 wherein
R is selected from: -NHCSNH(CH₂)₂NHCSNH-, -NHCSNH(CH₂)₃NHCSNH-, -NHCSNH(CH₂)₄NHCSNH-,
and salts and solvates thereof.

It is preferred that the compound according to the invention is selected from the group comprising or consisting of 1,4-Di[3-(3-isothiocyanatophenyl)thioureido]butane, 1-isothiocyanato-4-[2-(4-isothiocyanatophenyl)ethyl]]benzene, 1-amino-4-[[4-[[4-chloro-6-[(3-sulfophenyl)amino]-1,3,5-triazin-2-yl]amino]-3-sulfophenyl]amino]-9,10-dioxoanthracene-2-sulfonic acid. These compounds are all inhibitors of P2Y₆.

A particular preferred embodiment of the invention refers to the compound 1,4-Di[3-(3-isothiocyanatophenyl)thioureido]butane (also called MRS2578) for use in the treatment of diseases related to energy balance and carbohydrate metabolism and homeostasis. Said inhibitor of P2Y₆ has a molecular weight of 472.67 and can be described by the following formula:

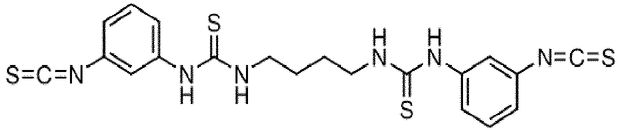

1,4-Di[3-(3-isothiocyanatophenyl)thioureido]butane (MRS2578) is a potent P2Y₆ receptor antagonist with IC₅₀ of 37 nM. 1,4-Di[3-(3-isothiocyanatophenyl)thioureido]butane (MRS2578) selectively blocks P2Y₆ receptor activity versus activity at P2Y₁, P2Y₂, P2Y₄ or P2Y₁₁ receptors.

A further preferred embodiment of the invention refers to the compounds described by the following formula: 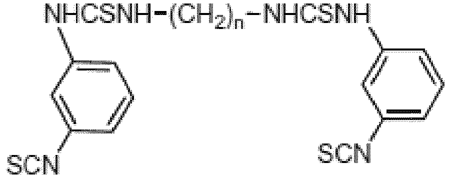 with n=4 or n=3.

Another embodiment of the invention refers to the compounds of formula I or II selected from: 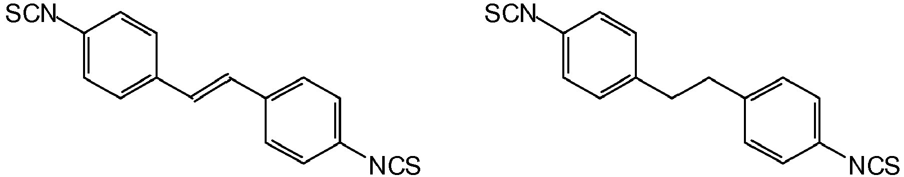 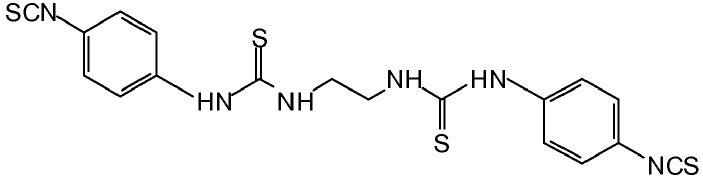 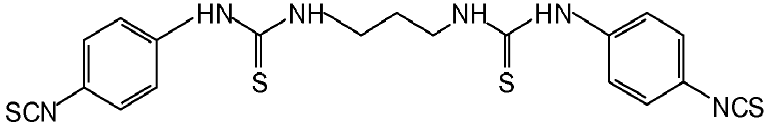 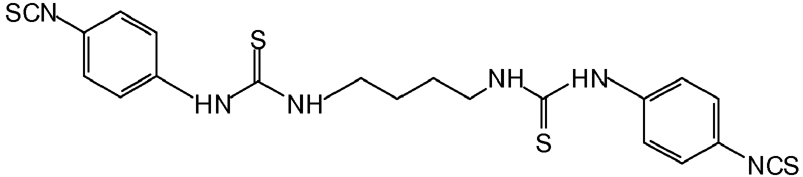 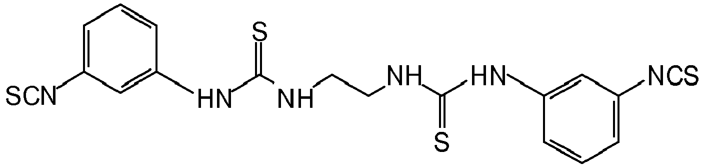 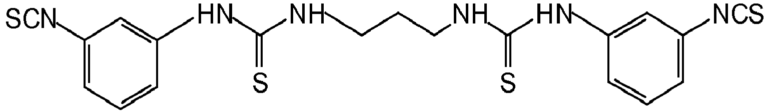 and

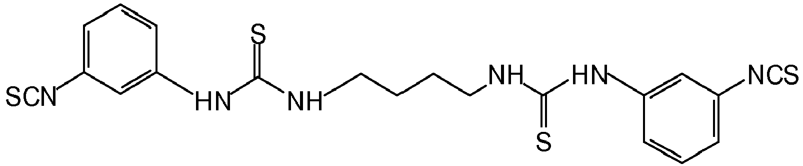

In the context of the present invention, the term "antibody" covers monoclonal antibodies, polyclonal antibodies, multispecific antibodies (e.g. bispecific antibodies) formed from at least two antibodies, antibody fragments and derivates thereof as long as they exhibit the desired activity. The antibody may be an IgM, IgG, e.g. IgG1, IgG2, IgG3 or IgG4. Antibody fragments comprise a portion of an antibody, generally the antigen binding or variable region of the intact antibody. Examples of antibody fragments include Fab, Fab', F(ab') 2 and Fv fragments, diabodies, single chain antibody molecules and multispecific antibody fragments. For therapeutic purposes, particularly for the treatment of humans, the administration of chimeric antibodies, humanized antibodies or human antibodies is especially preferred. The antibodies according to the invention may be coupled to a labeling group, particularly for diagnostic applications, e.g. the detection of diseases related to energy balance and carbohydrate metabolism and homeostasis. Examples for suitable labeling groups such as fluorescent groups are known in the art.

A compound acting on protein level may be an aptamer, i.e. an oligonucleic acid, a peptide molecule or a combination thereof that specifically binds to the target polypeptide. Aptamers are mostly short single stranded DNA- or RNA-molecules, which can bind a specific molecule because of their 3D-structure. Aptamers may be prepared by chemical synthesis and selected by a systematic evolution of ligands due to exponential enrichment as known by the person skilled in the art. An oligonucleic acid aptamer may have a sequence length of between about 20-100 nucleotides, preferably about 25-75, more preferably about 30-50 nucleotides. A peptide aptamer generally consists of a variable peptide loop attached at both ends to a protein scaffold. The variable loop length is between 5 and 50, preferably about 10-30, and more preferably about 10-20 amino acids. An aptamer according to the invention may be coupled to a labeling group, particularly for diagnostic applications. Furthermore, particularly for therapeutic applications, the aptamer may be coupled to an effector group, e.g. a cytotoxic group such as toxin.

In a further embodiment, the compound according to the invention is directed against a target nucleic acid, i.e. regulator acting on the nucleic acid level. Preferably, the compound is a nucleic acid compound, e.g. RNAi inducing molecules like siRNA, miRNA, anti-sense oligonucleotide, ribozyme, a precursor or a combination thereof. In a preferred embodiment of the invention, the regulator is an inhibitor of the expression of a target nucleic acid, which preferably acts by down-regulation or knock-down of the target. Down-regulation or knock-down of the target results preferably in a reduction of the steady state level of the target mRNA or polypeptide by at least factor 2, 5, 10 or more, or in a disappearance of the target from the cell.

A RNAi-inducing molecule may refer to a nucleic acid molecule, wherein at least one polynucleotide strand of said nucleic acid molecule has a sequence which is sufficiently complementary to a target RNA, preferably to a target mRNA, in order to effect its processing, i.e. its decomposition. In order to have an RNAi-inducing effect, it is necessary that the complementarity between the RNAi-inducing molecule and a region of the target RNA is sufficient, in order to effect a hybridization and a subsequent processing. For example, the complementarity is at least 80%, preferably at least 90% and most preferably at least 99%, whereby the 5'- and/or 3'-ends as well as the overhangs of an RNAi-effector molecule may also contain nucleotides, which are not complementary to the target RNA.

SiRNA (small interfering RNA or short interfering RNA or silencing RNA) used according to the invention is a double-strand of RNA and/or nucleotide analogues with 3' overhangs on at least one end, preferably either ends. Each RNA strand of the double-strand has a 5' phosphate group and a 3' hydroxyl group. Preferably, each RNA strand of the double strand is 19 to 30 nucleotides long, more preferably 20 to 28 nucleotides and most preferably 21 to 23 nucleotides. The 3' overhang on the end of a RNA strand is preferably 2 nucleotides long. In a particular preferred embodiment the siRNA double-strand consists of two 21 nucleotides long RNA strands each having a 2 nucleotides long 3' overhang. SiRNA molecules further refer to single-stranded RNA-molecules having a length of 19-30 nucleotides, preferably 20-28 nucleotides and particularly having a length of 21-23 nucleotides, whereby the single-stranded RNA molecule is for at least 80%, preferably for at least 90% and more preferably for more than 99% complementary to a sequence of a target RNA, in particular of a target mRNA, and a binding of siRNA to the target RNA effects a sequence specific decrease. Preferably, siRNA molecules have overhangs of 1-3 nucleotides on the 3' end. Methods for obtaining siRNA molecules are known to the person skilled in the art.

MiRNA is a single- or double-stranded RNA molecule of 19-30, preferably 20-28, and more preferably 21-23 nucleotides in length, which can regulate gene expression. MiRNA is generally synthesized at first as a precursor, which is then processed to the major form having a sequence which is at least partially complementary to messenger RNA of a target molecule according to the invention.

An antisense oligonucleotide may be a single, double, or triple-stranded DNA, RNA, PNA (peptide nucleic acid) or a combination thereof (e.g. hybrids of DNA and RNA strands) having a length of between about 10-100, preferably 20-50, and more preferably 20-30 nucleotides in length, which can interfere with mRNA targets by hybrid formation and therefore inhibit translation of said mRNA.

Ribozymes are catalytic RNAs possessing a well defined structure that enables them to catalyze a chemical reaction. Apart from naturally occurring ribozymes they can be made artificially and be tailored to interact with nucleic acids and proteins. Ribozymes are also preferred modulators for inhibition or activation of the preferred kinases in the present invention.

Precursor molecules, e.g. precursor molecules of siRNA and/or miRNA may be a substrate for the siRNA/miRNA-biogenesis-apparatus of the target cell. This comprises, for example, RNA precursor molecules such as double-stranded RNA (dsRNA) or short hairpin RNA-molecules (shRNA), which are processed by enodribonucleases such as Drosha and/or Pasha to siRNA-molecules or miRNA-molecules, respectively. Dicer is another endoribonuclease that cleaves double-stranded RNA and pre-microRNA (miRNA) into siRNA about 20-25 nucleotides long, usually with a two-base overhang on the 3' end. Dicer catalyzes the first step in the RNA interference pathway and initiates formation of the RNA-induced silencing complex (RISC). The RISC complex with a bound siRNA recognizes complementray mRNA molecules and degrades them, resulting in substantially decreased levels of protein translation and effectively turning off the gene. DsRNA-molecules or short hairpin RNA-molecules (shRNA) having a length of more than 27 nucleotides, preferably more than 30 up to 100 nucleotides or longer, and mostly preferred dsRNA-molecules having a length of 30-50 nucleotides, can be used.

Further precursor molecules according to the invention may be DNA constructs encoding dsRNA, shRNA, siRNA and/or miRNA, whereby the coding elements are controlled by regulatory elements allowing an expression of dsRNA, shRNA, siRNA and/or miRNA in the target cell. Examples for such control elements are polymerase II promoters or polymerase III promoters such as, for example, U6 or H1.

SiRNA, miRNA, ribozymes and antisense oligonucleotides may be coupled with a labeling group, e.g. for diagnostic purposes or may be coupled with an effector molecule known in the art and they may be applied to a target cell by any technique which is known to a person skilled in the art, such as transfection of exogenous siRNA, miRNA, ribozyme and antisense oligonucleotide or of an appropriate vector, e.g. viral or non-viral, producing a single transcript which can be processed into a functional siRNA, miRNA, ribozyme or antisense oligonucleotide.

A compound acting on the nucleic acid level as described above may exhibit analogs of one or more nucleotides within its nucleotide sequence. Said nucleotide analogs may, for example, increase the structural stability of the RNA molecule or the stability towards ribonucleases. Ribonucleotide analogs are well known to the person skilled in the art and are modified compared to the original RNA molecules by base modification, sugar modification, e.g. modification of the 2'-OH group of ribose and/or phosphate backbone modifications.

Diseases related to energy balance and carbohydrate metabolism and homeostasis refer to diseases and conditions characterized by pathological disorders of the metabolism. They are mainly characterized by enzyme defects and abnormalities in the regulating system leading to a pathological enrichment of substrates, lack of metabolic products, failure of producing energy, of regeneration of cellular constituents, of elimination of metabolic products, and of maintenance of homeostasis. They can be acquired or be a genetic disease. Diseases related to energy balance and carbohydrate metabolism and homeostasis as used herein are particularly, but are not limited to, obesity and diabetes. Carbohydrate metabolism denotes the various biochemical processes responsible for the formation, breakdown and interconversion of carbohydrates in living organisms, wherein the most important carbohydrate is glucose. The hormone insulin is the primary regulatory signal in animals; if present, it causes many tissue cells to take up glucose from the circulation, causes some cells to store glucose internally in the form of glycogen, causes some cells to take in and hold lipids, inhibits de novo glucose synthesis in some cells (liver, kidney) and in many cases controls cellular electrolyte balances and amino acid uptake as well. Diseases of the carbohydrate metabolism refer to diseases and conditions characterized in pathophysiological alterations in the metabolism of one or more carbohydrates. It is preferred if the disease of the carbohydrate homeostasis and metabolism is selected of one disease of the group comprising or consisting of obesity, diabetes mellitus, lactose intolerance, fructose intolerance, galactosemia, glycogen storage disease, diabetic ketoacidosis, hyperosmolar coma and hypoglycemia. Particularly preferred are obesity, type 2 diabetes and related complications selected from cardiovascular diseases, hepatic steatosis and lipid disorders.

Another aspect of the present invention refers to a compound for activation of P2Y purinoceptor 6 polypeptide or upregulation or modification for advanced transcriptional activity of a nucleic acid encoding P2Y purinoceptor 6, upregulation of P2Y purinoceptor 6 signaling pathway for the treatment of diseases related to energy balance and carbohydrate metabolism and homeostasis such as for gaining or maintaining weight, anorexia nervosa, cancer cachexia, underweight, treatment of underweight by newborns, treatment of underweight by people in extreme need of care.

In another embodiment of the present invention the compound is a small molecule, an RNA molecule, an siRNA molecule, an miRNA molecule, or a precursor thereof, a polypeptide or a ribozyme.

In a further embodiment of the present invention, the compound for the treatment of diseases related to energy balance and carbohydrate metabolism and homeostasis such as for gaining or maintaining weight, anorexia nervosa, cancer cachexia, underweight, treatment of underweight by newborns, treatment of underweight by people in extreme need of care is a small molecule, selected from the general formula **(III)** 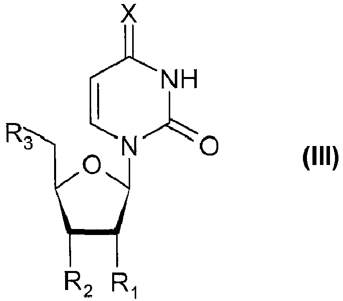 or salts and solvates thereof, wherein X represents =O or =S, R₁ represents -H, -OH, -OCHO, -OCOCH₃, -OCOC₂H₅, -OCOC₃H₇, -OCO-cyclo-C₃H₅, -OCOCH(CH₃)₂, -OCOC(CH₃)₃, -OCOC₄H₉, -OCOC₅H₁₁, -OCOCH(CH₃)-C₃H₇, -OCO-CH(CH₃)-C₂H₅, -OCOCH(CH₃)-CH(CH₃)₂, -OCOC(CH₃)₂-C₂H₅, -OCOCH₂-C(CH₃)₃, -OCO-C(CH₃)₃, -OCOCH(C₂H₅)₂, or -OCOC₂H₄-CH(CH₃)₂, and R₂ and R₃ represent independently of each other -OH, -OCHO, -OCOCH₃, -OCOC₂H₅, -OCOC₃H₇, -OCO-cyclo-C₃H₅, -OCOCH(CH₃)₂, -OCOC(CH₃)₃, -OCOC₄H₉, -OCOC₅H₁₁, -OCOCH(CH₃)-C₃H₇, -OCO-CH(CH₃)-C₂H₅, -OCOCH(CH₃)-CH(CH₃)₂, -OCOC(CH₃)₂-C₂H₅, -OCOCH₂-C(CH₃)₃, -OCO-C(CH₃)₃, -OCOCH(C₂H₅)₂, or -OCOC₂H₄-CH(CH₃)₂.

It is particular preferred that said small molecule of general formula **(III)** represents uridine preferably with (S) conformation of the ribose moiety, triacetyluridine or 4-thiouridine, even more preferably wherein the compound of general formula **(III)** represents uridine preferably with (S) conformation of the ribose moiety.

In another embodiment of the present invention, the compound for the treatment of diseases related to energy balance and carbohydrate metabolism and homeostasis such as for gaining or maintaining weight, anorexia nervosa, cancer cachexia, underweight, treatment of underweight by newborns, treatment of underweight by people in extreme need of care is a small molecule, selected from the general formula **(IV)** 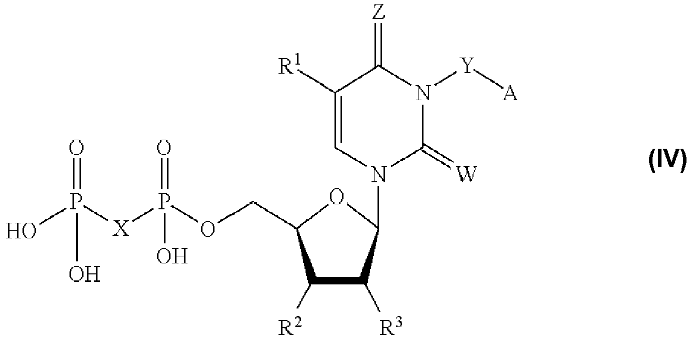 or a salt thereof,
wherein:
A is 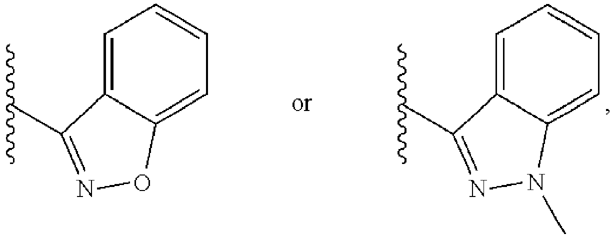 and wherein A is optionally further substituted with one or more R⁷;
X is selected from -O-, -S-, -N(R⁵)-, -CH₂-, -C₂H₄-, -C₃H₆-, -C(CH₃)₂-, and is independently and optionally substituted with one or more R⁴;
Y is a bond or -CH₂, -C₂H₄, -C₃H₆-, -C(CH₃)₂-, -C₄H₈-, -CH₂-C(CH₃)₂-, -CH(CH₃)-C₂H₄-, -C₅H₁₀, -CH(CH₃)-C₃H₆-, -CH₂-CH(CH₃)-C₂H₄-, -CH(CH₃)-C(CH₃)₂-, -C(CH₃)₂-C₂H₄-, -CH(C₂H₅)-, -C₂H₄-CH(CH₃)-, and is independently and optionally substituted with one or more R⁴;
Z and W are each independently selected from =O, =S, =N(R⁵), and =N-OR⁵;
R₁ is selected from:
   - H, halogen, -OR⁵, -CN, -CF₃, -OCF₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂₋C₃H₇, -C(CH₃)₂-CH(CH₃)₂, -C₂H₄-C(CH₃)₃, and -CH(CH₃)-C(CH₃)₃, and is optionally substituted with one or more R⁷;
R² and R³ are independently of each other selected from -OR⁵, -SR⁵, -NR⁵R⁶ and -OC(O)R⁵;
each occurrence of R⁴ is independently selected from:
   halogen, -OR⁵, -NO₂, -CN, -CF₃, -OCF₃, -R⁵, 1,2-methylenedioxy, 1,2-ethylenedioxy, -N(R⁵)₂, -SR⁵, -SOR⁵, -SO₂R⁵, -SO₂N(R⁵)₂, -SO₃R⁵, -C(O)R⁵, -C(O)C(O)R⁵, -C(O)CH₂C(O)R⁵, -C(S)R⁵, -C(S)OR⁵, -C(O)OR⁵, -C(O)C(O)OR⁵, -C(O)C(O)N(R⁵)₂, -OC(O)R⁵, -C(O)N(R⁵)₂, -OC(O)N(R⁵)₂, -C(S)N(R⁵)₂, -(CH₂)₀₋₂NHC(O)R⁵, -N(R⁵)N(R⁵)COR⁵, -N(R⁵)N(R⁵)C(O)OR⁵, -N(R⁵)N(R⁵)CON(R⁵)₂, -N(R⁵)SO_{2R}⁵, -N(R⁵)SO₂N(R⁵)², -N(R⁵)C(O)OR⁵, -N(R⁵)C(O)R⁵, -N(R⁵)C(S)R⁵, -N(R⁵)C(O)N(R⁵)², -N(R⁵)C(S)N(R⁵)₂, -N(COR⁵)COR⁵, -N(OR⁵)R⁵, -C(=NH)N(R⁵)₂, -C(O)N(OR⁵)R⁵, -C(=NOR⁵)R⁵, -OP(O)(OR⁵)₂, -P(O)(R⁵)₂, -P(O)(OR⁵)₂, or -P(O)(H)OR⁵);
each occurrence of R⁵ is independently selected from:
   -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂₋C₃H₇, -C(CH₃)₂-CH(CH₃)₂, -C₂H₄-C(CH₃)₃, -CH(CH₃)-C(CH₃)₃, 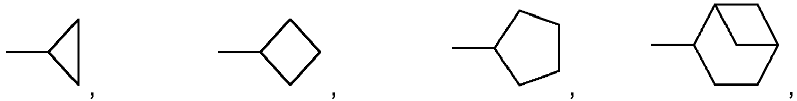 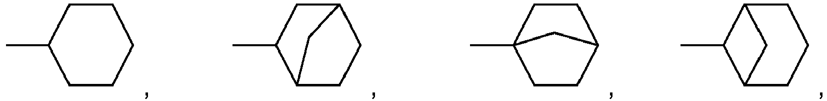 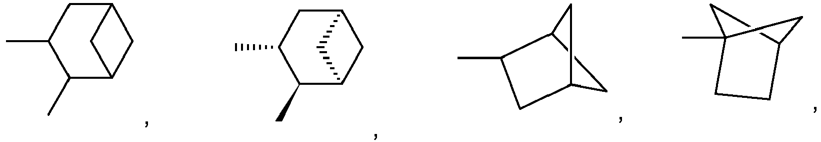 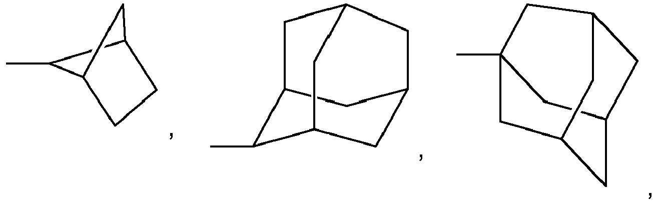 2-thienyl, 3-thienyl, 2-furanyl, 3-furanyl, 2-oxazolyl, 3-oxazolyl, 4-oxazolyl, 2-thiazolyl, 3-thiazolyl, 4-thiazolyl, 1-pyrazolyl, 3-pyrazolyl, 4-pyrazolyl, 5-pyrazolyl, 1-imidazolyl, 2-imidazolyl, 4-imidazolyl, 5-imidazolyl, phenyl, 1-naphthyl, 2-naphthyl, 2-pyridyl, 3-pyridyl, 4-pyridyl, 2-pyrimidinyl, 4-pyrimidinyl, 5-pyrimidinyl, 2-pyrazinyl, 3-pyridazinyl, 4-pyridazinyl, 1,3,5-triazin-2-yl, 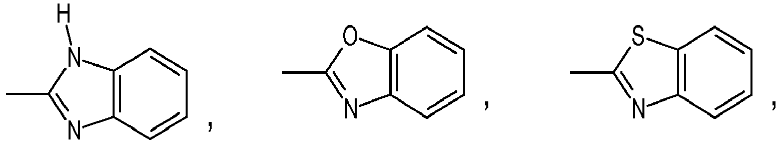 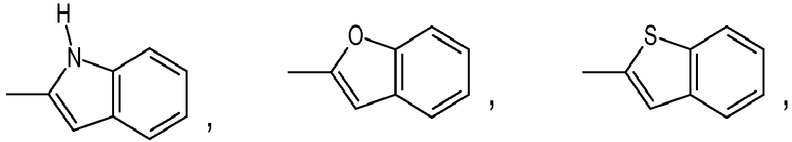 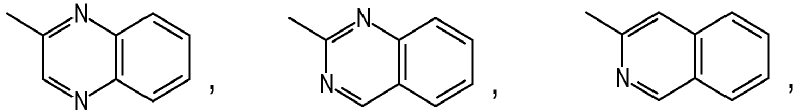 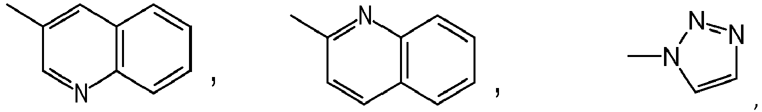 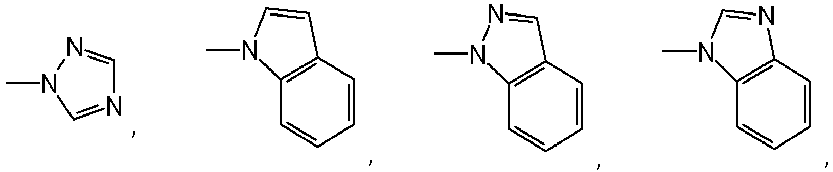 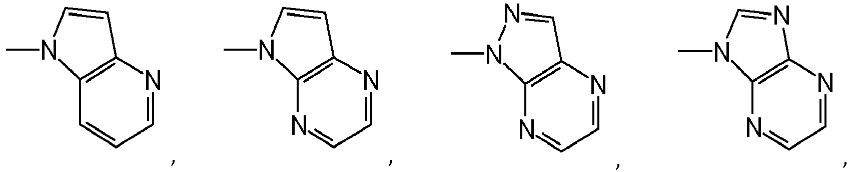 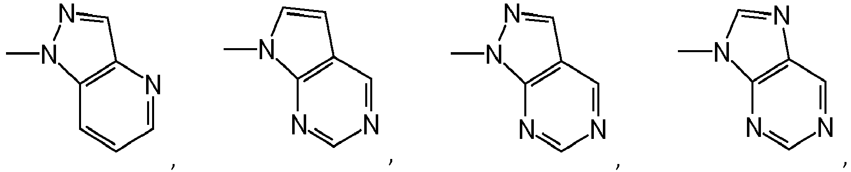 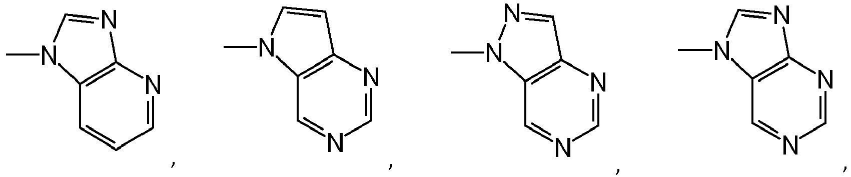 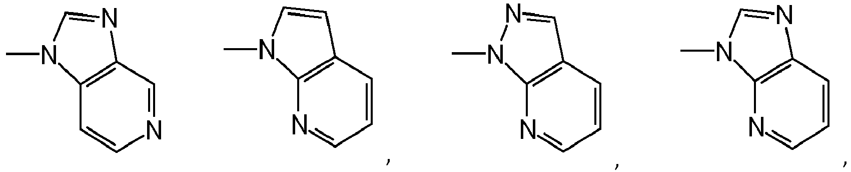 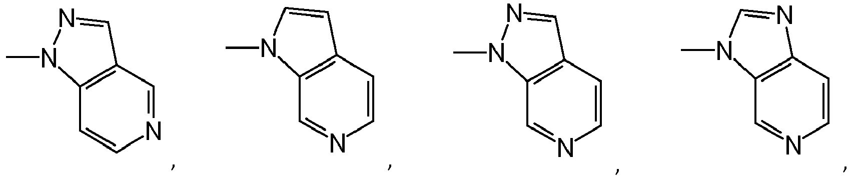 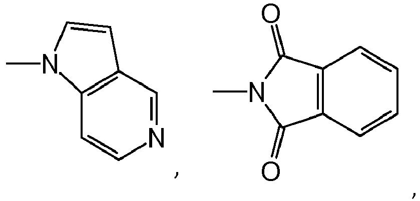
   wherein two R⁵ groups bound to the same atom optionally form a 3- to 6-membered aromatic or non-aromatic ring having up to 3 heteroatoms independently selected from N, O, S, SO, or SO₂, wherein said ring is optionally fused to a (C6-C10)aryl, (C5-C10)heteroaryl, (C3-C10)cycloalkyl, or a (C3-C10)heterocycloyl;
   and wherein each R⁵ group is independently and optionally substituted with one or more R⁷;
   R⁶ is selected from:
   - R⁵, -C(O)R⁵, -C(O)OR⁵, -C(O)N(R⁵)₂ and -S(O)₂R⁵;
each occurrence of R⁷ is independently selected from:
halogen, -OR⁸, -NO₂, -CN, -CF₃, -OCF₃, -R⁸, oxo, thioxo, 1,2-methylenedioxy, 1,2-ethylenedioxy, -N(R⁸)₂, -SR⁸, -SOR⁸, -SO₂R⁸, -SO₂N(R⁸)₂, -SO₃R⁸, -C(O)R⁸, -C(O)C(O)R⁸, -C(O)CH₂C(O)R⁸, -C(S)R⁸, -C(S)OR⁸, -C(O)OR⁸, -C(O)C(O)OR⁸, -C(O)C(O)N(R⁸)₂, -OC(O)R⁸, -C(O)N(R⁸)₂, -OC(O)N(R⁸)₂, -C(S)N(R⁸)₂, -(CH₂)₀₋₂NHC(O)R⁸, -N(R⁸)N(R⁸)COR⁸, -N(R⁸)N(R⁸)C(O)OR⁸, -N(R⁸)N(R⁸)CON(R⁸)₂, -N(R⁸)SO₂R⁸, -N(R⁸)SO₂N(R⁸)₂, -N(R⁸)C(O)OR⁸, -N(R⁸)C(O)R⁸, -N(R⁸)C(S)R⁸, -N(R⁸)C(O)N(R⁸)₂, -N(R⁸)C(S)N(R⁸)₂, -N(COR⁸)COR⁸, -N(OR⁸)R⁸, -C(=NH)N(R⁸)₂, -C(O)N(OR⁸)R⁸, -C(=NOR⁸)R⁸, -OP(O)(OR⁸)₂, -P(O)(R⁸)₂, -P(O)(OR⁸)₂, or -P(O)(H)(OR⁸); and
each occurrence of R⁸ is independently selected from:
-H -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂_C₃H₇, -C(CH₃)₂-CH(CH₃)₂, -C₂H₄-C(CH₃)₃, and -CH(CH₃)-C(CH₃)₃.

It is further preferred that said small molecule of general formula **(IV)** represents: 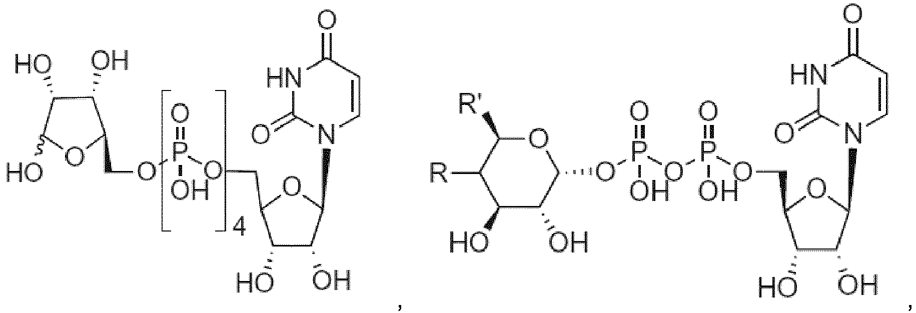 wherein R = OH and R'= COOH, or CH₂OH,

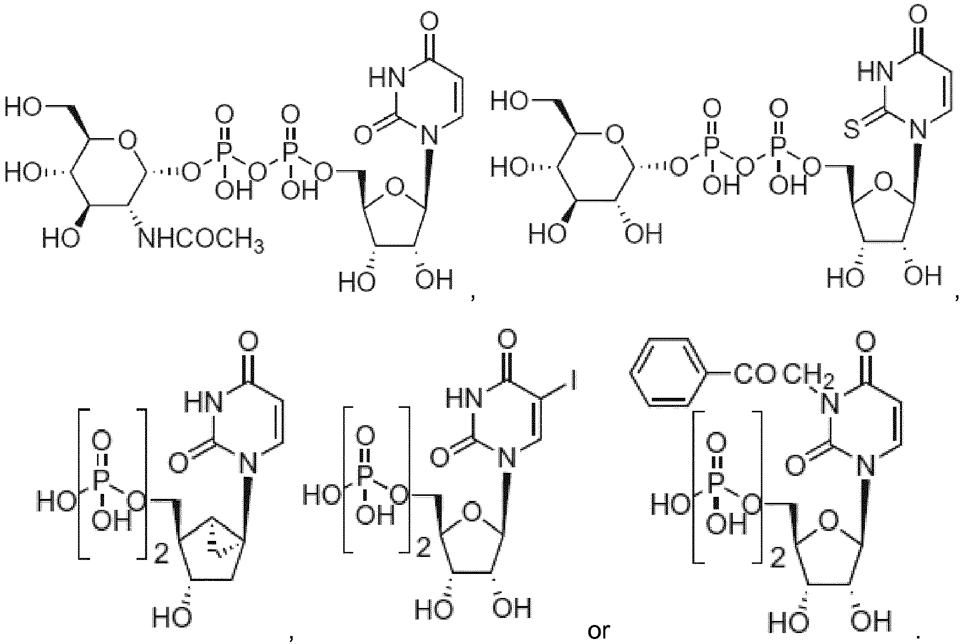

It is further preferred that said small molecule is selected from the group consisting of: 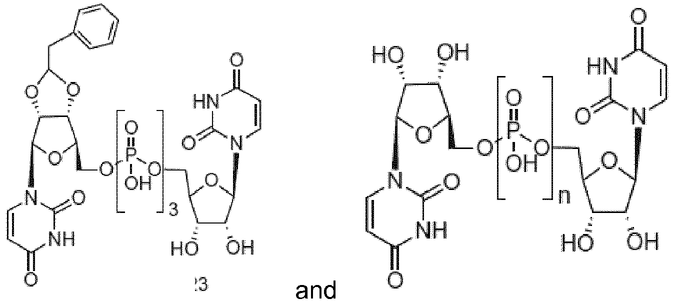 wherein n=3.

Uridine is attractive for therapeutic use because of its low toxicity. Major limiting factors in increasing uridine doses are fever and diarrhea. Oral administration of uridine can increase uridine level in plasma as well as in brain.

In another embodiment of the present invention, the compound is a uridine precursor, uridine, uridine derivative, UDP or an activator of UDP synthesis, any regulator to increase UDP half-life or an activator of P2Y₆ pathway for use in a therapy for increasing food intake and fat mass.

In yet another embodiment of the present invention the compound is uridine wherein the ribose moiety is preferably in the (S) conformation or uridine diphosphate (UDP) derivatives, which are derived from a similar compound by some chemical or physical process. However, the ribose moiety of the uridine, UDP or UDP-derivative is preferably in the (S) conformation. Furthermore, uridine, UDP, or UDP-derivatives are preferably for use in a therapy increasing food intake and fat mass. In yet another embodiment of the present invention the compound is an activator of P2Y₆ pathways or a compound that increases the synthesis of UDP, preferably for use in a therapy increasing food intake and fat mass.

The term "uridine precursor" as used herein refers to any chemical compound preceding uridine in the uridine biosynthesis or may be converted into uridine when administered. The term "uridine derivative" as used herein refers to any compound that is derived from uridine by some chemical or physical process and can bind to P2Y purinoceptor 6 or activate the P2Y₆ pathways.

Uridine is the natural precursor of UDP in the CNS. Thus, when administering uridine, the level of UDP in the hypothalamus is increased. UDP directly activates orexigenic NPY/AgRP neurons via activation of P2Y₆, which is in perfect accordance with its ability to promote feeding.

Increase of food intake is suitable for patients if they have lost a lot of weight due to illness, which may be because of problems with reduced appetite or impaired food resorption. Thus, it is preferred that uridine is used (administered) for therapies increasing food intake in the treatment of cancer cachexia, treatment of anorexia nervosa, treatment of underweight, treatment of underweight by newborns, and treatment of underweight by people in extreme need of care.

Enhancement of food intake is also desirable in animal breeding. Therefore another aspect of the present invention refers to uridine for use as stimulant of animal weight. The present invention refers also to the use of uridine precursor, uridine derivative, an activator of UDP synthesis, any regulator to increase UDP half-life or an activator of P2Y₆ pathway in animal feed, preferably as stimulant of food intake.

Furthermore, the present invention provides a method for stimulating increased rate of growth, greater amount of growth and greater feed efficiency in domesticated animals, said method comprising:
a) activation of P2Y purinoceptor 6 polypeptide or
b) upregulation or modification for advanced transcriptional activity of a nucleic acid encoding P2Y purinoceptor 6,
c) upregulation of P2Y purinoceptor 6 signaling pathway
d) activation of uridine transport across the blood brain barrier,
e) activation or increase of UDP synthesis in the CNS,
f) prevention of UDP degradation

The term "domesticated animals" refers in particular to cattle, pigs, sheep and other fattening animals.

The invention relates generally to veterinary pharmaceutical compositions and formulations that
a) stimulate P2Y purinoceptor 6 polypeptide or
b) upregulate a nucleic acid encoding P2Y purinoceptor 6,
c) prevente degradation of a nucleic acid encoding P2Y purinoceptor 6,
d) activation of uridine transport across the blood brain barrier,
e) activation or increase of UDP synthesis in the CNS,
f) prevention of UDP degradation
g) upregulate P2Y purinoceptor 6 signaling pathway.

Another embodiment of the present invention relates to compounds that increase UDP synthesis and thereby stimulate animal food intake.

The present invention concerns a method of stimulating increased rate of growth, greater amount of growth and greater feed efficiency in food and fattening animals which comprises providing to such animals biodegradable and non-biodegradable compressed tablets loaded with
a) an activator for P2Y purinoceptor 6 polypeptide or
b) an activator for upregulation of a nucleic acid encoding P2Y purinoceptor 6,
c) an inhibitor of degradation of a nucleic acid encoding P2Y purinoceptor 6,
d) an activator for uridine transport across the blood brain barrier,
e) an activator for or enhancer for UDP synthesis in the CNS,
f) an inhibitor for UDP degradation
g) an activator for upregulation of P2Y purinoceptor 6 signaling pathway.

The method of the present invention provides advantages over methods known in the art such as, inter alia, increased weight gain.

For use as a medicament, the inventive compound may be formulated as a pharmaceutical composition. Therefore still another aspect of the present invention deals with pharmaceutical compositions comprising at least one compound as defined according to the invention as an active ingredient. The medication comprising a compound as described according to the invention can be formulated to be suitable for any known dosage form. The composition may be administered by one dose per day or may be divided up to several doses. The effective amount of the active agent, i.e. the compound according to the invention, in the composition may be determined by the skilled person without undue burden depending on the kind of active agent and the kind of conditions to be treated. For example, about 1 µg/kg to 15 mg/kg of a regulator may be administered to a human patient, e.g. by one or more separate administrations or by continuous infusion. A typical daily dosage may range from about 1 µg/kg to about 100 mg/kg or more, depending on the factors such as age, gender and weight of the person to be treated etc.

A pharmaceutical composition as defined in the present invention may be further administered as a part of a combination therapy or combinatorial therapy. In the context of the present invention, "combination therapy" or "combinatorial therapy" also refers to the simultaneous administration of two or more active agents, wherein at least one of these agents is a compound according to the present invention. The two or more active agents may be administered simultaneously in one single pharmaceutical composition or more than one pharmaceutical composition, wherein each composition comprises at least one active agent.

The invention relates also to pharmaceutical compositions comprising or consisting of at least one compound according to the invention for the treatment of a disease of the energy balance and carbohydrate metabolism and homeostasis. In another embodiment the pharmaceutical compositions comprises an effective amount of at least one inventive compound, and at least one pharmaceutically acceptable carrier, excipient, binders, disintegrates, glidents, diluents, lubricants, coloring agents, sweetening agents, flavoring agents, preservatives, solvent or the like. The pharmaceutical compositions of the present invention can be prepared in a conventional solid or liquid carrier or diluents and a conventional pharmaceutically-made adjuvant at suitable dosage level in a known way.

According to the invention, the inventive compound or the pharmaceutical composition can be used for the treatment of diseases related to energy balance and carbohydrate metabolism and homeostasis to modulate or regulate food intake, body weight and glucose levels.

The inventive pharmaceutical composition is formulated to be compatible with its intended route of administration. Administration forms include, for example, pills, tablets, film tablets, coated tablets, capsules, liposomal formulations, micro- and nano-formulations, powders and deposits. Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain the compound according to the present invention. Intravenous, intramuscular and oral applications are preferred forms of administration in the present invention, wherein oral application is particularly preferred.

The present invention also includes artificial mammalian milk as well as mammalian milk substitutes as a formulation for oral administration of the inventive compound to newborns, toddlers, infants either as pharmaceutical preparations, and/or as dietary food supplements.

The inventive compound can also be administered in form of its pharmaceutically active salts. Suitable pharmaceutically active salts comprise acid addition salts and alkali or earth alkali salts. For instance, sodium, potassium, lithium, magnesium or calcium salts can be obtained.

The pharmaceutical compositions according to the present invention will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. Other suitable formulations are gels, elixirs, dispersible granules, syrups, suspensions, creams, lotions, solutions, emulsions, suspensions, dispersions, and the like. Suitable dosage forms for sustained release include tablets having layers of varying disintegration rates or controlled release polymeric matrices impregnated with the active components and shaped in tablet form or capsules containing such impregnated or encapsulated porous polymeric matrices. The pharmaceutical compositions may be comprised of 5 to 95% by weight of the inventive compound.

As pharmaceutically acceptable carrier, excipient and/or diluents can be used lactose, starch, sucrose, cellulose, magnesium stearate, dicalcium phosphate, calcium sulfate, talc, mannitol, ethyl alcohol (liquid filled capsules).

Suitable binders include starch, gelatine, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavouring agents and preservatives may also be included where appropriate. Some of the terms noted above, namely disintegrants, diluents, lubricants, binders and the like, are discussed in more detail below.

Additionally, the compounds or regulators of the present invention may be formulated in sustained release form to provide the rate controlled release of any one or more of the components or active ingredients to optimize the therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.

Also included are solid form preparations being intended to be converted to liquid form preparations for either oral or parenteral administration, shortly before use. Such liquid forms include solutions, suspensions and emulsions.

The inventive compound may also be delivered transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art. Oral gels refer to the active ingredients dispersed or solubilized in a hydrophilic semi-solid matrix.

Powders for constitution refer to powder blends containing the active ingredients and suitable diluents which can be suspended in water or juices. One example for such an oral administration form for newborns, toddlers and/or infants is a human breast milk substitute which is produced from milk powder and milk whey powder, optionally and partially substituted with lactose.

Human breast milk is a complex fluid, rich in nutrients and in non-nutritional bioactive components. It contains all of the nutrients needed by the newborn baby. These include the metabolic components (fat, protein, and carbohydrates), water, and the raw materials for tissue growth and development, such as fatty acids, amino acids, minerals, vitamins, and trace elements.

The compounds of the present invention may be components of artificial mother milk formulations in order to increase food intake of newborns having underweight. Artificial mother milk formulations or mother milk substitutes of the present invention are preferably prepared by adding to a mother milk formulation including commercially available mother milk formulations especially in powder form of the compound of the present invention. The inventive compound is preferably added in an amount of 3 - 100 µg compound or per 100 ml (commercially available) mother milk formulation, more preferably in an amount of 5 - 70 µg / 100 ml and most preferably in an amount of 10 - 40 µg / 100 ml mother milk formulation.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropylmethylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate and polyethylene glycols. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

Liquid form preparations include solutions, suspensions and emulsions. Water or water-propylene glycol solutions for parenteral injections may be mentioned as an example. Liquid form preparations may also include solutions for intranasal administration.

Techniques for the formulation and administration of the compound of the present invention may be found in "Remington's Pharmaceutical Sciences" Mack Publishing Co., Easton PA. A suitable composition comprising the compound mentioned herein may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

Still another aspect of the present invention relates to the use of the inventive compound as a dietary supplement. That dietary supplement is preferably for oral administration and especially but not limited to administration to newborns, toddlers, and/or infants. A dietary supplement is intended to supplement the diet. The "dietary ingredients" in these products may in addition include: vitamins, minerals, herbs or other botanicals, amino acids, and substances such as enzymes, organ tissues, glandulars, and metabolites. Dietary supplements may be manufactured in forms such as tablets, capsules, softgels, liquids, or powders.

The invention further relates to a method for screening for a compound for treatment of diseases related to energy balance and carbohydrate metabolism and homeostasis, the method comprising
a) contacting a test compound with a P2Y purinoceptor 6 polypeptide,
b) detecting the binding of said test compound to the P2Y purinoceptor 6 polypeptide, and
c) determining the activity of the P2Y purinoceptor 6 polypeptide in the presence of said test compound,
wherein instead of polypeptides nucleic acids encoding the polypeptides are used and the expression rate is determined instead of the activity, preferably wherein the test compound is RNA or a peptide or an antibody or a small molecule.

The term "contacting" as used herein refers to the step wherein the test compound dissolved in water or a mixture of water and a water-soluble organic solvent such as DMSO, acetone, ethanol, methanol, tetrahydrofuran, DMF, ethylacetate, and isopropanol, is mixed with a P2Y purinoceptor 6 polypeptide in water so that the test compound could bind to the P2Y purinoceptor 6 polypeptide in case the test compound has any affinity to the P2Y purinoceptor 6 polypeptide.

The screening method of the present invention apparently consists of three steps. The term test compound may be any of the potential compounds listed above. The contacting of the test compound with at least one P2Y purinoceptor 6 polypeptide can happen e.g. in the form of a compound library, in physiological or non-physiological solution, or solid phase systems, however a liquid environment is preferred. The conditions and the time need to be sufficient to allow the test compound to bind to the polypeptide. The method is normally carried out in solution at room temperature and at a suitable pH value normally between pH 5 and 9, all parameters being easily selected by a skilled person. The P2Y₆ polypeptide can be obtained by purification from primary human cells, cell lines or from cells being transfected with expression constructs which contain the nucleic acid sequences encoding a P2Y₆ polypeptide.

In a preferred screening method of the present invention the test compound is not contacted with a P2Y purinoceptor 6 polypeptide but with nucleic acids encoding the polypeptides and the expression rate in regard to these nucleic acids is determined instead of the polypeptide activity. It is preferred that the test compound is RNA or a peptide or an antibody or a small molecule.

The nucleic acid sequences encoding a P2Y₆ polypeptide can be obtained by cloning the relevant gene, amplification of the cDNAs or chemical synthesis of the nucleic sequences. For the expression of the corresponding polypeptides the nucleic acid sequences can be inserted into expression vectors, such as recombinant bacteriophage, plasmid, or cosmid DNA expression vectors.

The term binding refers to an interaction between the test compound and one or more a P2Y₆ polypeptide or the nucleic acids encoding a P2Y₆ polypeptide. For binding to a protein, the binding interaction is dependent upon the presence of a particular structure of the test compound, e.g. a binding site of an antagonist or an agonist recognized by the receptor. For binding of compounds to nucleic acids, test compounds need to have a complementary sequence to the nucleic acids, or fit into certain secondary or tertiary structures of the nucleic acids.

The binding of the test compounds to the polypeptide or nucleic acids can be checked by any convenient method known in the art. A separation step may be included to separate bound from unbound components. To check whether the test compound has been bound by the polypeptide or nucleic acid, it is advantageous if the test compound and/or the P2Y₆ polypeptide is labeled for direct detection (radioactivity, luminescence, fluorescence, optical or electron density etc.) or indirect detection (e.g., epitope tag such as the FLAG, V5 or *myc* epitopes, an enzyme tag such as horseradish peroxidase or luciferase, a transcription product, etc.). The label may be bound to a substrate, to the proteins employed in the assays, or to the test compound being the candidate pharmacological agent. The binding of a test compound can also be conveniently checked if one of the components is immobilized on a solid substrate.

Protein-DNA interactions can be for instance checked by gel shift or band shift assays or electrophoretic mobility shift assays (EMSA), which is based on the observation that complexes of protein and DNA migrate through a non-denaturing polyacrylamide gel more slowly than free DNA fragments.

The interactions between peptides or proteins, respectively, can be investigated by various methods, which include, but are not limited to, protein binding microarray, antibody microarrays, protein chips, a variety of assays and UV-crosslink experiments.

In all methods to identify compounds that regulate (activate or inhibit) the expression and signaling activity (via the corresponding G-protein) of the P2Y₆, the expression level and signaling activity are compared to those detected in the absence of the test compound. The present invention is related particularly to the identification of compounds, which have regulatory activity on the signaling activity of the P2Y₆ receptor. Consequently, it is particularly the inhibition or activation of expression and signaling activity that is measured.

The inhibition or activation of nucleic acids on the mRNA-level encoding the P2Y₆ polypeptides can be checked by investigating the expression of the polypeptides by quantitative methods, e.g. Western blot or enzyme-linked immune-adsorbent assay (ELISA). A way to quantify the protein expression is further the measuring of fusion proteins, wherein the polypeptides of the invention are fused to proteins or protein fragments, which are easy to quantify, like fluorescent proteins. The inhibition or activation of DNA and thus the production of mRNA can be checked by mRNA-quantification. Levels of mRNA can be quantitatively measured by Northern blotting. Another way is the reverse transcription quantitative polymerase chain reaction (RT-PCR followed by qPCR).

The inhibition or activation of the polypeptides on the protein-level can be investigated by measuring their activity. The determination of the activity of a polypeptide/protein/enzyme depends on its specificity. Consequently, the activity of G-protein coupled receptors is measured in assays, wherein a substrate of the signaling pathway downstream of the receptor and its G-protein is quantified such as cAMP. Measuring the radioactivity of phosphate being part of cAMP is one possibility.

P2Y₆ polypeptides are also useful in competition binding assays in methods designed to discover compounds that interact with the receptor (e.g. binding partners and/or ligands). Thus, a compound is exposed to a P2Y₆ polypeptide under conditions that allow the compound to bind or to otherwise interact with the polypeptide. A known ligand such as UDP is also added to the mixture. If the test compound interacts with the receptor, it competes with UDP for the binding to the receptor. Thus, the amount of bound/unbound UDP is changed in presence of the test compound compared to the absence of the test compound. This type of assay is particularly useful in cases to investigate if compounds bind to the receptor at its UDP-binding site.

The invention is further related to a method for treatment of diseases related to energy balance and carbohydrate metabolism or homeostasis, preferably obesity, diabetes mellitus type 2 and associated diseases comprising:
administering a subject in need thereof a therapeutically effective amount of at least one compound for:
   a) inhibition of P2Y purinoceptor 6 polypeptide,
   b) inactivation, degradation, downregulation or intercalation of a nucleic acid encoding P2Y purinoceptor 6 or
   c) downregulation of P2Y purinoceptor 6 signaling pathway.

The invention is further related to a method for treatment of diseases related to energy balance and carbohydrate metabolism or homeostasis, preferably obesity, diabetes mellitus type 2 and associated diseases comprising:
administering a subject in need thereof a therapeutically effective amount of at least one compound for:
   a) inhibition of UDP synthesis,
   b) inhibition of UDP synthesis in the CNS,
   c) inhibition of uridine transport to the CNS,
   d) inhibition of uridine transport across the blood brain barrier,
   e) acceleration or increase of UDP degradation,
   f) acceleration or increase of UDP degradation in the CNS, or
   g) inhibition of enzymes important for the synthesis of UDP in the CNS.

In regard to said method, the term "a subject in need thereof" refers in regard to said method to a patient having the risk to develop diseases related to energy balance and carbohydrate metabolism or homeostasis, in particular to develop obesity or a diabetes type 2 or it refers to a patient that has already developed and, thus, suffers from obesity or diabetes mellitus type 2. The term "a therapeutically effective amount" refers to an amount of a compound sufficient to at least reduce in-vivo food intake and symptoms of diseases related to energy balance and carbohydrate metabolism or homeostasis such as obesity, diabetes type 2 and associated complications.

Additionally the present invention is related to a method of treating diseases related to energy balance and carbohydrate metabolism and homeostasis, preferably obesity or diabetes mellitus type 2 comprising administering to a patient in need of such treatment an effective amount of a compound for
a) inhibition of P2Y purinoceptor 6 polypeptide,
b) inactivation, degradation, downregulation or intercalation of a nucleic acid encoding P2Y purinoceptor 6, or
c) downregulation of P2Y purinoceptor 6 signaling pathway.

Furthermore, the present invention is related to a method of optimizing therapeutic efficacy for treatment of diseases related to energy balance and carbohydrate metabolism and homeostasis, preferably obesity or diabetes mellitus type 2, comprising:
(a) Administering an inhibitor of P2Y purinoceptor 6 polypeptide to a subject having said diseases related to energy balance and carbohydrate metabolism and homeostasis; or
(b) Inactivating, degrading, down regulating or intercalating a nucleic acid encoding P2Y purinoceptor 6 in said subject having said diseases related to energy balance and carbohydrate metabolism and homeostasis.

Further the present application relates to a compound for
(i) inhibition of P2Y purinoceptor 6 polypeptide,
(ii) inactivation, degradation, downregulation or intercalation of a nucleic acid encoding P2Y purinoceptor 6 or
(iii) downregulation of P2Y purinoceptor 6 signaling pathway
for the manufacture of a medicament for the treatment of a disease related to energy balance or carbohydrate metabolism and homeostasis such as obesity, type 2 diabetes and associated complications.

Preferably, a compound or regulator according to the invention is used in medicine and more preferred for the treatment of a disease related to energy balance and carbohydrate metabolism and homeostasis such as obesity and diabetes mellitus type 2.

A compound known to affect and in particular to decrease the expression and/or activity of the polypeptides of P2Y₆ purinoceptor can be used for the treatment of diseases related to energy balance and carbohydrate metabolism and homeostasis, preferably obesity, diabetes mellitus, more preferably diabetes mellitus type 2, and preferably for obesity by administration of said compound(s) within pharmaceutical compositions as outlined above.

The compounds or compositions according to the invention are useful for each single disease of the group comprising or consisting of diseases related to energy balance and carbohydrate metabolism and homeostasis, preferably obesity and diabetes type 2.

The invention is further related to a method for treatment of a disease being related to energy balance and carbohydrate metabolism and homeostasis such as cancer cachexia, underweight, treatment of underweight by newborns, treatment of underweight by people in extreme need of care comprising:
administering a subject in need thereof a therapeutically effective amount of at least one compound for:
   a) activation of P2Y purinoceptor 6 polypeptide,
   b) activation, upregulation of a nucleic acid encoding P2Y purinoceptor 6,
   c) upregulation of P2Y purinoceptor 6 signaling pathway,
   d) activation of Uridine transport across the blood brain barrier,
   e) activation or increase of UDP synthesis in the CNS,
   f) prevention of UDP degradation, or
   g) prevention of UDP degradation in the CNS.

The invention is further related to a method for treatment of a disease being related to energy balance and carbohydrate metabolism or homeostasis such as cancer cachexia, underweight, treatment of underweight by newborns, treatment of underweight by people in extreme need of care comprising:
administering a subject in need thereof a therapeutically effective amount of at least one compound for:
   a) activation of UDP synthesis,
   b) activation of uridine transport to the CNS, or
   c) activation of enzymes important for the synthesis of UDP in the CNS.

The term "a subject in need thereof" refers in regard to said method to a patient with cancer cachexia, underweight, or a newborn with underweight, or people in extreme need of care with underweight. The term "a therapeutically effective amount" refers in regard to said method to an amount of a compound sufficient to at least increase in-vivo food intake and fat mass.

Preferably, a compound or regulator described herein is used in medicine and more preferred for the treatment of a disease related to energy balance and carbohydrate metabolism and homeostasis such as cancer cachexia, underweight, treatment of underweight by newborns, treatment of underweight by people in extreme need of care.

A compound known to affect and in particular to increase the expression and/or activity of the polypeptides of P2Y₆ purinoceptor can be used for the treatment of cancer cachexia, underweight, treatment of underweight by newborns, treatment of underweight by people in extreme need of care by administration of the inventive compound(s) within pharmaceutical compositions as outline above.

The compounds or compositions according to the invention are useful for each single disease of the group comprising or consisting of cancer cachexia, underweight, underweight by newborns, underweight by people in extreme need of care.

One embodiment of the invention relates to a method for the diagnosis of diabetes type 2, comprising: providing a sample, preferably a blood or serum sample, from a subject suspected of having diabetes type 2; and detecting the level of uridine in the sample. The uridine level measured is compared to standardized uridine level or to previous values of the respective patient. Thereby elevated uridine level is indicative for diabetes type 2.

**Table 1 Sequence identities of the target genes and target proteins**

| **SeqID No.** | **Sequence Name** | **NCBI Accession** PRI 15-FEB-2014 |
|---|---|---|
| 1 | P2Y₆ cDNA | NM_001277204 |
| | | Version: NP_001264133.1 |
| 2 | P2Y₆ protein | NP_001264133 |
| | | Version: NP_001264133.1 |
| 3 | P2RY6 gene | NC_000011.10 |
| | | Chr 11:_72.98 - 73.01 Mb |

The embodiments in the description and the following examples are provided by way of illustration of the invention and are not included for the purpose of limiting the invention. The variations and changes of the invention which are obvious to a person skilled in the field and solutions equivalent to embodiments described herein fall within the scope of protection of the patent claims.

### Examples

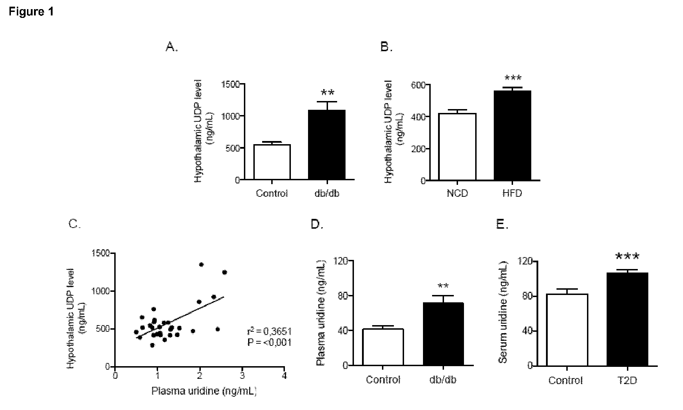

The inventors found that hypothalamic UDP levels are abnormally regulated upon obesity and diabetes. Indeed, hypothalamic UDP levels are increased in nutritionally as well as genetically-induced obese/diabetic mice (respectively the diet-induced obese mice fed a high-fat diet (HFD) as compare to normal chow diet-fed (NCD) mice and the genetically obese/diabetic mice) (Figure 1A-1B). The increased-UDP levels associated with obesity and diabetes seems to be primarily driven by increased circulating uridine levels, as plasma uridine levels are increased in obese/diabetic mice and hypothalamic UDP levels positively correlates with circulating uridine (Figure 1C-1D). Interestingly, the inventors found that this increased circulating levels of uridine is also present in human diagnosed with T2D, suggesting the existence of convergent uridine/UDP regulation across species and therefore, that this work may also be relevant in human (Figure 1 E).

The inventors could show that elevated circulating uridine appears to be associated with diabetes in human as serum uridine levels are increase in patients diagnosed with type 2 diabetes (Figure 1 E). That correlation suggests a particular, but not limited to, utilization of uridine level for diagnosis of disease related to energy balance and carbohydrate metabolism and homeostasis, preferably diabetes mellitus type 2.

### Example 1

Investigation of the regulation of P2Y₆ and its ligand uridine diphosphate (UDP) in the hypothalamus of obese and diabetic mice was performed. For that purpose nutritionally as well as genetically-induced obese/diabetic mice were used. Diet-induced obese mice, C57BL/6N males (purchased from Charles River) received high-fat diet (HFD) or control normal chow diet (NCD) starting at 8 weeks of age until sacrifice (20 weeks). HFD (purchased from Sniff Diets) contains (in calories from): 21% carbohydrates, 19% protein, and 60% fat (D12492-I) and NCD (purchased from Sniff Diets) contains 70% carbohydrates, 20% protein, and 10% fat (D12450B). Mice homozygous for the mutated form of the leptin receptor isoform b (db/db mice) and their control littermates were purchased from the Jackson Laboratory. Mice were housed as described above, briefly in individual cages under specific pathogen-free conditions, maintained in a temperature-controlled room and provided ad libitum access to water and, unless stated otherwise, standard laboratory chow-diet.

HFD- and NCD-fed mice (n= 9-10 per group) as well as db/db and control mice (n= 7-10 per group) were sacrificed by decapitation. Trunk blood was collected for plasma preparation and hypothalami were quickly dissected and frozen. Prior to uridine measurement, mice plasma and human sera were deproteinized using perchloric acid treatment (70 %; Sigma). Hypothalami were homogenized in 70% methanol solution and lyophilized. Hypothalamic UDP and circulating uridine levels were measured by UPLC as described by Denzel et al., 2014, Cell 156, 1167-1178.

It was found that UDP level was increased in the hypothalamus of HFD as well as db/db mice (Fig. 1A/1B), suggesting a critical role of circulating uridine in the regulation of hypothalamic UDP level. Supporting this finding, hypothalamic UDP level positively and significantly correlates with plasmatic uridine (Fig. 1C). In addition, elevated circulating uridine appears to be associated with diabetes in human as serum uridine level increase in patients diagnosed with type 2 diabetes (Fig. 1 E). The control and the type 2 diabetes (T2D) groups were matched regarding sex ratio, age and body weight, but differ from the diagnosis of type 2 diabetes (control, n= 160; T2D, n=238).

### Example 2

To explore whether UDP plays a direct role on the central regulation of feeding behavior, the effect of intracerebroventricular administration (ICV) of UDP on spontaneous food intake in wild type mice fed with a normal chow diet was measured. Therefore, mice were anesthetized using Ketamine/Xylasine (138 mg/kg body weight and 6,9 mg/kg body weight, respectively). Guide cannulas (26 gauges) were stereotaxically inserted into the lateral ventricle (bregma -0,2 mm, midline 1.0 mm, dorsal surface -2,1 mm). Animals were allowed to recover for one week prior to experiments.

Prior to the onset of the dark phase, mice acutely received 2 µL ICV administration of UDP (1 µM, 10 µM and 30 µM; Sigma-Aldrich®) (n= 11-15 per group), MRS 2578 (1 µM, 10 µM; Sigma-Aldrich®) (n= 5-10 per group), or control solution (NaCl) (n= 9-27 per group). Spontaneous food intake was measured 2 and 4 hours after ICV administration from pre-weighed portions of food dispensed from the food rack. ICV administration of UDP increases food intake in a dose-dependent manner (Fig. 2A). UDP appears to be a strong orexigenic agent as administration of 30 µM UDP induces a 45% increase of spontaneous food intake.

In an opposite fashion, ICV administration of MRS2578, a non-competitive selective antagonist of P2Y₆ decreases food intake (Fig. 2B). This set of data shows that central UDP and P2Y₆ modulate feeding behavior and that inhibition of P2Y₆ is able to decrease food intake.

### Example 3

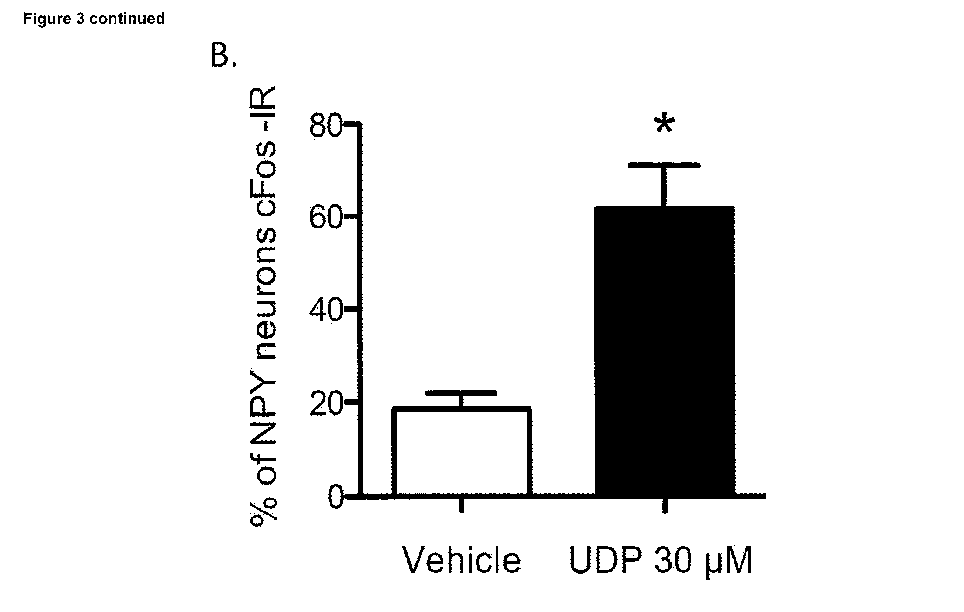

Assessment of the pattern of neuronal activation following UDP ICV administration was performed, using c-Fos-activation as an experimental readout. The number of c-Fos immunoreactive neurons expressing NPY was quantified by expression of the green-fluorescent protein (GFP) under control of the NPY promoter (NPY-GFP) (Fig. 3A). For the quantitative analysis of cell number, the numbers of cFos-, NPY- and cFos/NPY-immunoreactive cells in the ARH were manually counted using Image J analysis software (NIH). The average number of cells counted in two ARH hemisections from each mouse was used for statistical comparisons. UDP activates 60% of NPY-expressing neurons (Fig. 3B). Transgenic mice were used that selectively express the humanized renilla GFP (hrGFP) under control of the NPY-promoter (NPY-GFP mice). NPY-GFP male received ICV administration of UDP (2µL; 30 µM UDP) or control solution (2µL saline) (n= 3-4 per group), and were transcardially perfused 1 hour later with 4% paraformaldehyde. Brains were then processed for immunofluorescence as described above. Briefly, sections were incubated with goat anti-cFos antibody (1:500; Santa-Cruz) and primary antibody was localized using donkey anti-goat IgGs conjugated with Alexa 568 (1:200; Invitrogen).

### Example 4

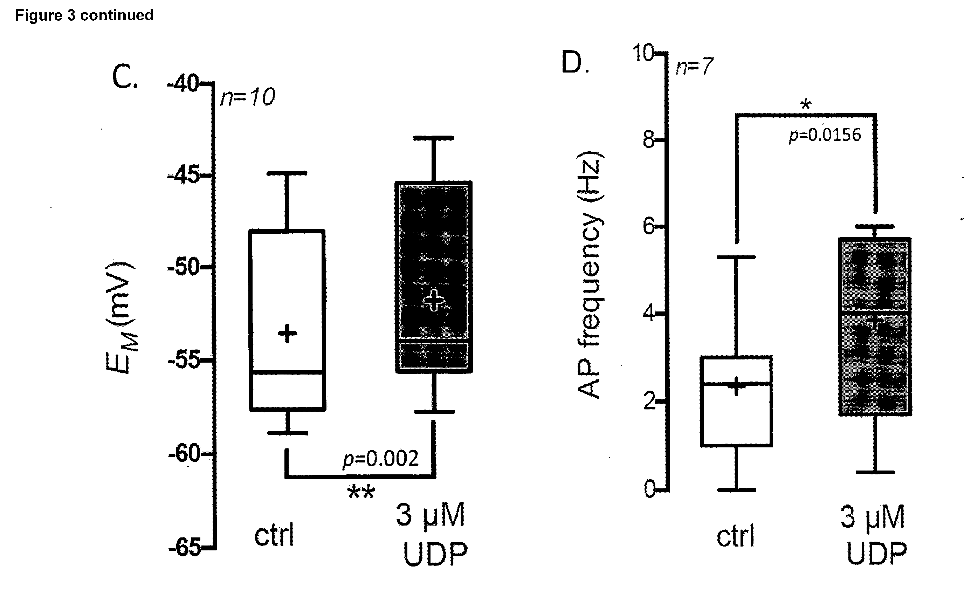

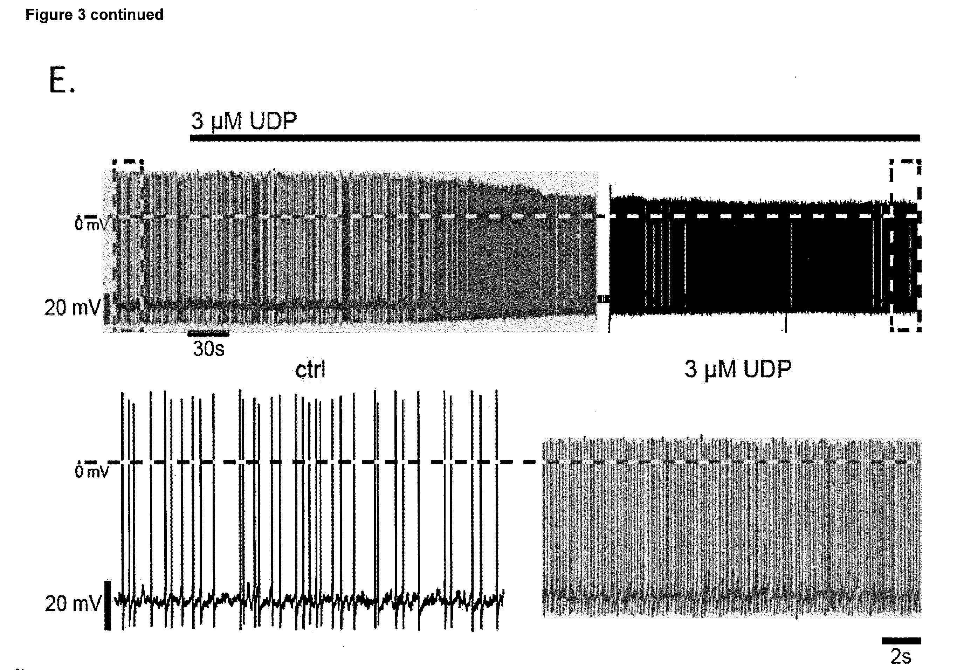

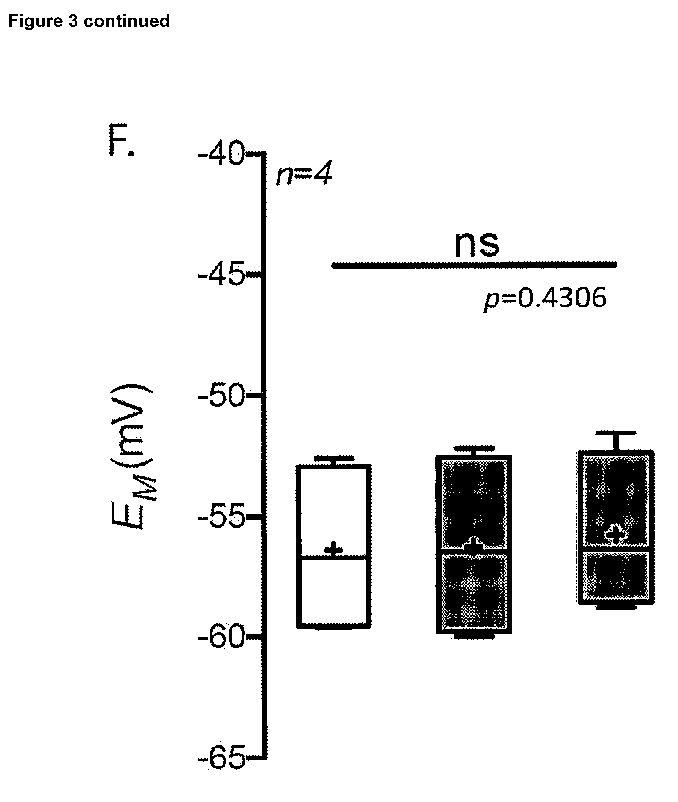

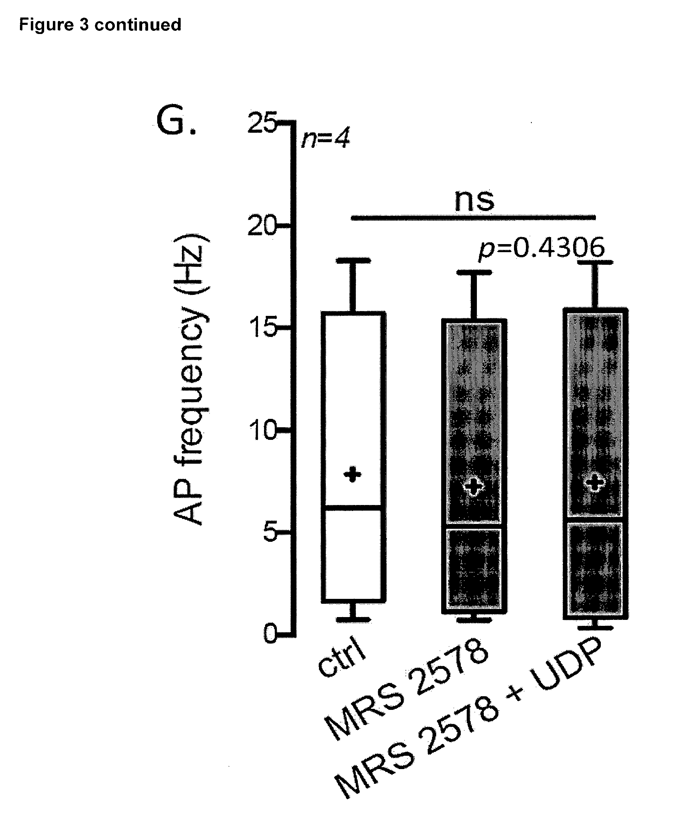

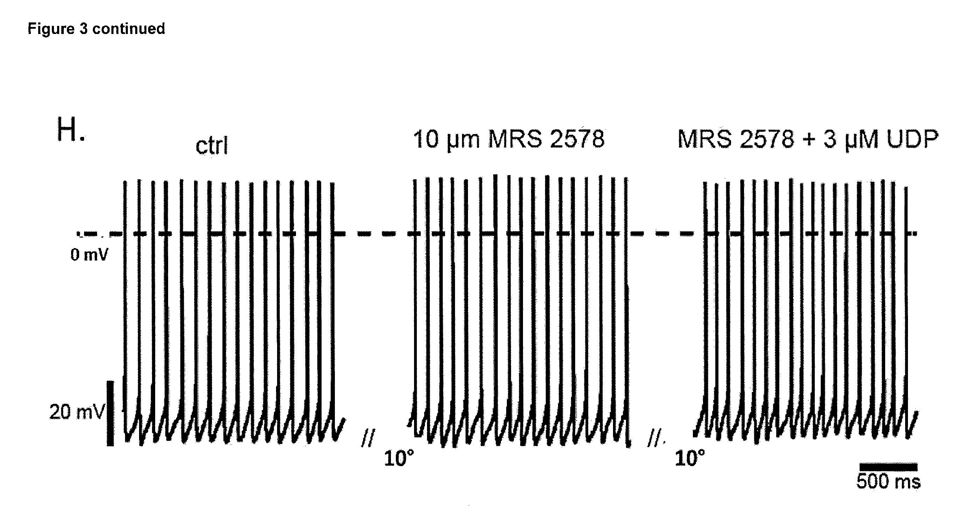

Electrophysiological parameters of synaptically isolated neurons were examined using patch-clamp recording. AgRP and NPY neurons were recorded from adults AgRP^{tdTomato} and NPY-GFP mice. The tomato reporter line (Rosa)26Sortm9(CAG-tdTomato)Hze mice were breed to AgRP-IRES-cre mice to obtain AgRP^{tdTomato}. Brains were sectioned and processed as described previously by Vogt et al., 2014, Cell 156, 495-509. All experiments have been conducted in synaptically isolated neurons with a dose of 3 µM UDP. UDP depolarization of the membrane potential and increase of the action potential frequency for synaptically isolated neurons is shown in Fig. 3C and 3D respectively. Electrophysiological parameter records of AgRP/NPY neurons upon UDP-induced activation are presented as well (Fig. 3E). For experiments with MRS2578, pre-treatment with MRS2578 (10 µM UDP) was applied prior to UDP application (3 µM) (n= 4-10 neurons per group). Pre-treatment of brain slices with MRS2578 abolished response to UDP (Fig. 3F/3G/3H), proving that UDP -induced depolarization of AgRP/NPY neurons is directly mediated via P2Y₆.

All animal procedures were conducted in compliance with protocols approved by local government authorities (Bezirksregierung Köln; district council Cologne).

### Statistics

All values were expressed as the means ± SEM. Statistical analyses were conducted using GraphPad PRISM (version 5.0d). Statistical significance was determined using unpaired two-tailed Student's t-tests or one-way analysis of variance (ANOVA) followed by a Bonferroni's poshoc test for experiments with more than two groups. For electrophysiology experiments, data are depicted as boxplots according to Tukey and statistics determined using non-parametric paired student t-test or one-way ANOVA (nonparametric; Friedman-Test). P < 0.05 was considered to be statistically significant. *p < 0.05, **p < 0.01, and ***p < 0.001 versus control and treated group.

### Description of the figures

- Figure 1:: Comparison of hypothalamic UDP level of genetically-induced obese/diabetic (db/db mice) mice and wild type mice (control) is shown (Fig. 1A). UDP level of obese mice fed a high-fat diet (HFD) compared to normal chow diet-fed mice (NCD) (Fig. 1 B). Increase of UDP levels in the hypothalamus of nutritionally-induced obese/diabetic mice (HFD) is shown. HFD as well as db/db mice show increase of UDP levels in the hypothalamus (Fig. 1A/B). Correlation of hypothalamic UDP level with plasmatic uridine level (Fig. 1C) reveals that hypothalamic UDP level positively and significantly correlates with plasmatic uridine. Comparison of uridine level in plasma of db/db mice compared to control mice (Fig. 1 D) shows increase of plasma uridine level in db/db mice. Elevated circulating uridine was shown in human sera (Fig. 1 E). Comparison of human sera from patients diagnosed with type 2 diabetes (T2D) groups and control groups are shown. P < 0.05 was considered to be statistically significant, **p<0.01, and *** p< 0.001 versus control and treated group.
- Figure 2:: Effect of 2µL intracerebroventricular (ICV) administration of UDP (1 µM, 10 µM and 30 µM) and MRS2578 (1 µM and 10 µM), a non-competitive selective antagonist of P2Y₆, on spontaneous food intake was assessed. Control mice were ICV administrated with 2µL control solution (NaCl). A dose dependent increase in food intake relative to control is shown upon UDP administration. 30 µM UDP induces a 45% increase of spontaneous food intake (Fig. 2A). ICV administration of 10 µM MRS2578 decreases food intake significantly (Fig. 2B). All mice were fed with a normal chow diet.
- Figure 3:: Assessment of pattern of neuronal activation following UDP ICV administration, wherein mice expressing green-fluorescent protein (GFP) under control of NPY promoter (NPY-GFP) were used as experimental readout. Quantification of expression of GFP in c-Fos-immunoreactive NPY neurons after ICV administration of 2µL of UDP 30 µM UDP or control (2 uL, Saline) is shown (Fig. 3A). UDP activates 60% of NPY-expressing neurons (Fig. 3B). UDP depolarization of the membrane potential of synaptically isolated neurons (Fig. 3C) and increase of the action potential frequency of neurons (Fig. 3D) due to presence of UDP is shown. Electrophysiological parameter recordings of AgRP/NPY neurons upon UDP-induced activation (Fig. 3E). Pre-treatment of brain slices with MRS2578 (10µM) abolished response to UDP (3µM) (Fig. 3F/3G/3H), proving that UDP -induced depolarization of AgRP/NPY neurons is directly mediated via P2Y₆.

## Claims

1. A compound which regulates the activity of P2Y purinoceptor 6 signaling pathway for the treatment of diseases related to energy balance and carbohydrate metabolism and homeostasis.

2. The compound according to claim 1 for
a) inhibition of P2Y purinoceptor 6 polypeptide,
b) inactivation, degradation, downregulation or intercalation of a nucleic acid encoding P2Y purinoceptor 6,
c) downregulation of P2Y purinoceptor 6 signaling pathway,
d) inhibition of uridine transport across the blood brain barrier,
e) inhibition of UDP synthesis in the CNS, or
f) acceleration or increase of UDP degradation
for use in the treatment of diseases related to energy balance and carbohydrate metabolism and homeostasis.

3. The compound according to claim 1 or 2, wherein the disease related to energy balance and carbohydrate metabolism and homeostasis is selected from the group consisting of obesity, type 2 diabetes and related complications selected from cardiovascular diseases, hepatic steatosis, and lipid disorders.

4. The compound according to any one of claims 1 - 3, wherein the compound is
a) a small molecule,
b) an RNA molecule,
c) an siRNA molecule, an miRNA molecule, or a precursor thereof,
d) an antisense oligonucleotide,
e) an aptamer
f) a polypeptide,
g) an antibody, or
h) a ribozyme.

5. The compound according to any one of claims 1 - 4, wherein said compound is of general formula **(I)** 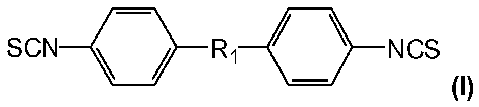 wherein
R₁ is selected from: trans-CH=CH-, -CH₂-CH₂-, -NHCSNH(CH₂)₂NHCSNH-, -NHCSNH(CH₂)₃NHCSNH-, and -NHCSNH(CH₂)₄NHCSNH-
or said compound is of general formula **(II)** 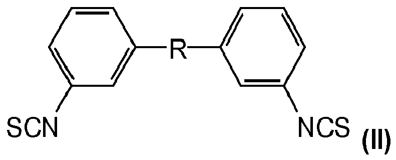 wherein
R is selected from: -NHCSNH(CH₂)₂NHCSNH-, -NHCSNH(CH₂)₃NHCSNH-, and -NHCSNH(CH₂)₄NHCSNH-,
or salts and solvates of compounds of general formula I and II.

6. The compound according to any one of claims 1 - 4, wherein said compound is selected from the group consisting of 1,4-di[3-(3-isothiocyanatophenyl)thioureido]butane, 1-isothiocyanato-4-[2-(4-isothiocyanato-phenyl)ethyl]benzene, and 1-amino-4-[[4-[[4-chloro-6-[(3-sulfophenyl)amino]-1,3,5-triazin-2-yl]amino]-3-sulfophenyl]amino]-9,10-dioxoanthracene-2-sulfonic acid.

7. The compound according to claim 1 for
a) activation of P2Y purinoceptor 6 polypeptide,
b) upregulation or modification for advanced transcriptional activity of a nucleic acid encoding P2Y purinoceptor 6,
c) upregulation of P2Y purinoceptor 6 signaling pathway,
d) activation of uridine transport across the blood brain barrier,
e) activation or increase of UDP synthesis in the CNS, or
f) prevention of UDP degradation
for use in a therapy for gaining or maintaining weight.

8. The compound according to claim 1 or 7, wherein the disease is selected from the group consisting of anorexia nervosa, cancer cachexia, underweight, treatment of underweight by newborns, and treatment of underweight by people in extreme need of care.

9. The compound according to any one of claims 7 or 8, wherein the compound is
a) a small molecule,
b) an RNA molecule,
c) an siRNA molecule, an miRNA molecule, or a precursor thereof,
d) an antisense oligonucleotide,
e) an aptamer
f) a polypeptide,
g) an antibody, or
h) a ribozyme.

10. The compound according to any one of claims 7 - 9, wherein said compound is selected from the general formula **(III)** 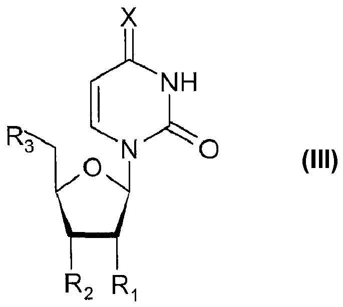 or salts and solvates thereof, wherein
X represents =O or =S,
R₁ represents -H, -OH, -OCHO, -OCOCH₃, -OCOC₂H₅, -OCOC₃H₇, -OCO-cyclo-C₃H₅, -OCOCH(CH₃)₂, -OCOC(CH₃)₃, -OCOC₄H₉, -OCOC₅H₁₁, -OCOCH(CH₃)-C₃H₇, -OCO-CH(CH₃)-C₂H₅, -OCOCH(CH₃)-CH(CH₃)₂, OCOC(CH₃)₂-C₂H₅, -OCOCH₂-C(CH₃)₃, -OCO-C(CH₃)₃, -OCOCH(C₂H₅)₂, or -OCOC₂H₄-CH(CH₃)₂,
R₂ and R₃ represent independently of each other -OH, -OCHO, -OCOCH₃, -OCOC₂H₅, -OCOC₃H₇, -OCO-cyclo-C₃H₅, -OCOCH(CH₃)₂, -OCOC(CH₃)₃, -OCOC₄H₉, -OCOC₅H₁₁, -OCOCH(CH₃)-C₃H₇, -OCO-CH(CH₃)-C₂H₅, -OCOCH(CH₃)-CH(CH₃)₂, -OCOC(CH₃)₂-C₂H₅, -OCOCH₂-C(CH₃)₃, -OCO-C(CH₃)₃, -OCOCH(C₂H₅)₂, or -OCOC₂H₄-CH(CH₃)₂, or the general formula **(IV)** 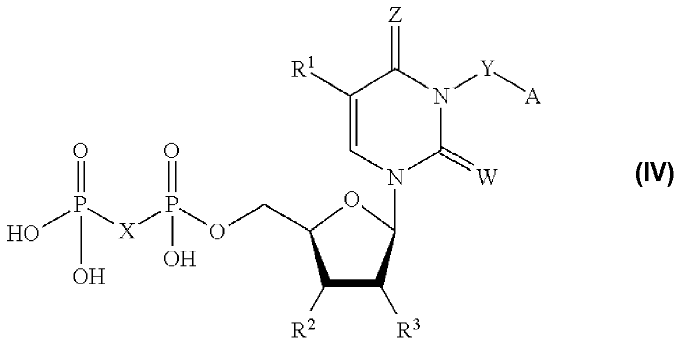 or salts and solvates thereof, wherein:
A is 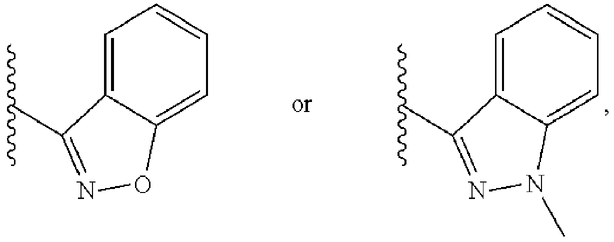
and wherein A is optionally further substituted with one or more R⁷;
X is selected from -O-, -S-, -N(R⁵)-, -CH₂-, -C₂H₄-, -C₃H₆-, -C(CH₃)₂, and can independently and optionally be substituted with one or more R⁴;
Y is a bond, -CH₂-, -C₂H₄-, -C₃H₆-, -C(CH₃)₂-, -C₄H₈-, -CH₂-C(CH₃)₂-, -CH(CH₃)-C₂H₄-, -C₅H₁₀-, -CH(CH₃)-C₃H₆-, -CH₂-CH(CH₃)-C₂H₄-, -CH(CH₃)-CH(CH₃)-, -C(CH₃)₂-C₂H₄-, -CH₂-C(CH₃)₂-, -C(C₂H₅)₂-, or -C₂H₄-C(CH₃)₂-, and can independently and optionally be substituted with one or more R⁴;
Z and W are each independently selected from =O, =S, =N(R⁵), and =N-OR⁵;
R¹ is selected from: -H, halogen, -OR⁵, -CN, -CF₃, -OCF₃ and -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂_C₃H₇, -C(CH₃)₂-CH(CH₃)₂, -C₂H₄-C(CH₃)₃, and -CH(CH₃)-C(CH₃)₃, and can optionally be substituted with one or more R⁷;
R² and R₃ are independently of each other selected from -OR⁵, -SR⁵, -NR⁵R⁶ and -OC(O)R⁵;
R⁴ is selected from: halogen, -OR⁵, -NO₂, -CN, -CF₃, -OCF₃, -R⁵, 1,2-methylenedioxy, 1,2-ethylenedioxy, -N(R⁵)₂, -SR⁵, -SOR⁵, -SO₂R⁵, -SO₂N(R⁵)₂, -SO₃R⁵, -C(O)R⁵, -C(O)C(O)R⁵, -C(O)CH₂C(O)R⁵, -C(S)R⁵, -C(S)OR⁵, -C(O)OR⁵, -C(O)C(O)OR⁵, -C(O)C(O)N(R⁵)₂, -OC(O)R⁵, -C(O)N(R⁵)₂, -OC(O)N(R⁵)₂, -C(S)N(R⁵)₂, -(CH₂)₀₋₂NHC(O)R⁵, -N(R⁵)N(R⁵)COR⁵, -N(R⁵)N(R⁵)C(O)OR⁵, -N(R⁵)N(R ⁵)CON(R⁵)₂₃ -N(R⁵)SO₂R⁵, -N(R⁵)SO₂N(R⁵)₂, -N(R⁵)C(O)OR⁵, -N(R⁵)C(O)R⁵, -N(R⁵)C(S)R⁵, -N(R⁵)C(O)N(R⁵)₂, -N(R⁵)C(S)N(R⁵)₂, -N(COR⁵)COR⁵, -N(OR⁵)R⁵, -C(=NH)N(R⁵)₂, -C(O)N(OR⁵)R⁵, -C(=NOR⁵)R⁵, -OP(O)(OR⁵)₂, -P(O)(R⁵)₂, -P(O)(OR⁵)₂, and -P(O)(H)OR⁵);
R⁵ is selected from: -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂₋C₃H₇, -C(CH₃)₂-CH(CH₃)₂, -C₂H₄-C(CH₃)₃, and -CH(CH₃)-C(CH₃)₃, 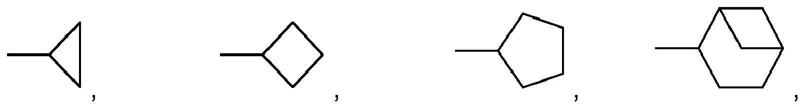 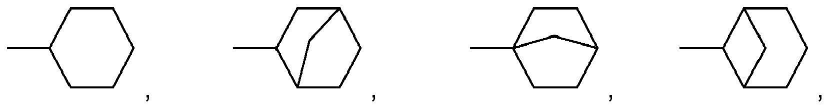 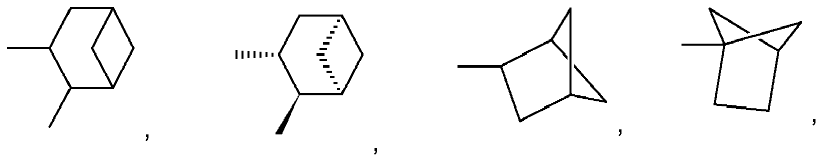 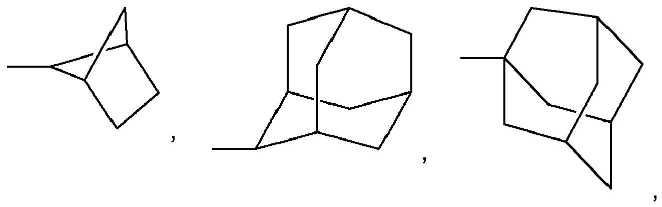 2-thienyl, 3-thienyl, 2-furanyl, 3-furanyl, 2-oxazolyl, 3-oxazolyl, 4-oxazolyl, 2-thiazolyl, 3-thiazolyl, 4-thiazolyl, 1-pyrazolyl, 3-pyrazolyl, 4-pyrazolyl, 5-pyrazolyl, 1-imidazolyl, 2-imidazolyl, 4-imidazolyl, 5-imidazolyl, phenyl, 1-naphthyl, 2-naphthyl, 2-pyridyl, 3-pyridyl, 4-pyridyl, 2-pyrimidinyl, 4-pyrimidinyl, 5-pyrimidinyl, 2-pyrazinyl, 3-pyridazinyl, 4-pyridazinyl, 1,3,5-triazin-2-yl, 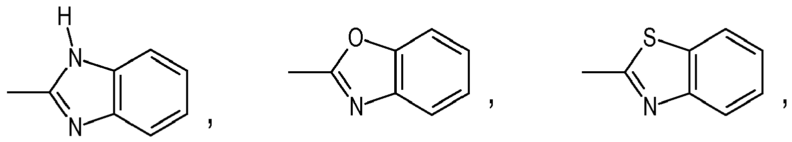 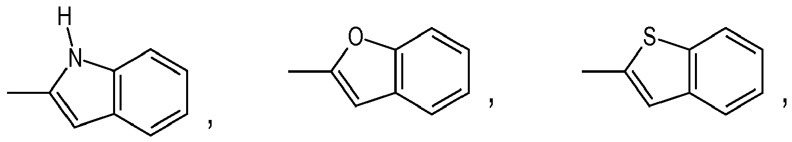 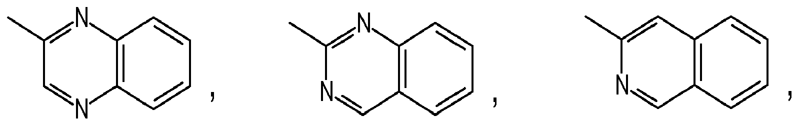 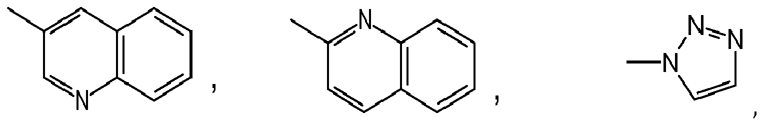 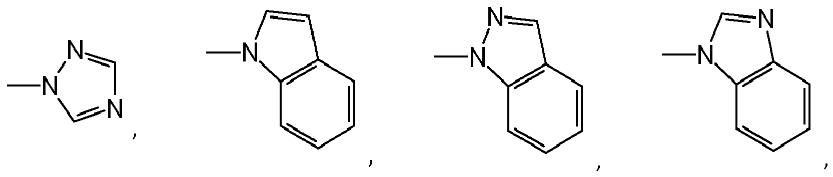 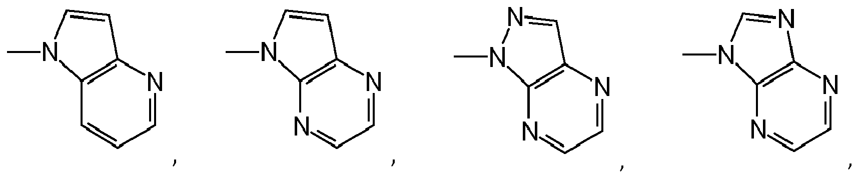 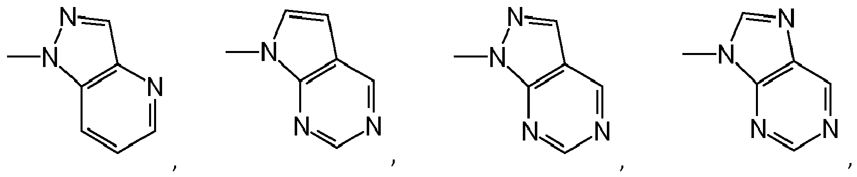 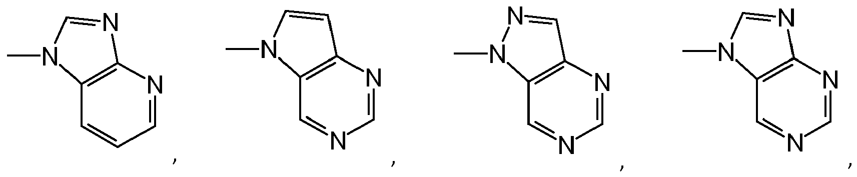 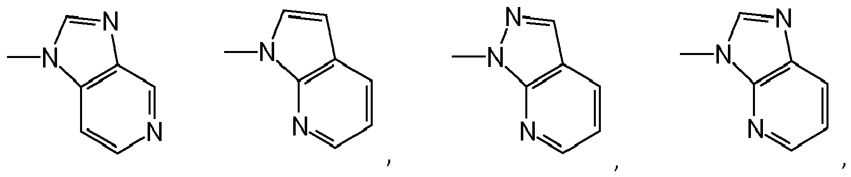 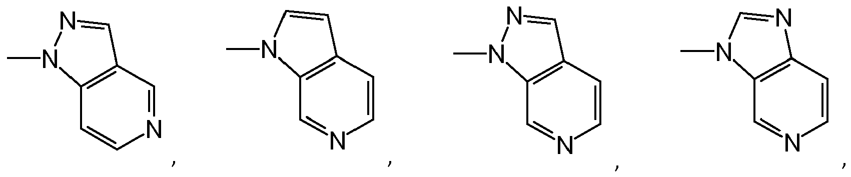 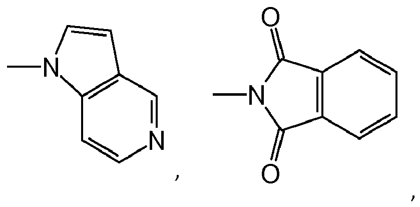
wherein two R⁵ groups bound to the same atom optionally form a 3- to 6-membered aromatic or non-aromatic ring having up to 3 heteroatoms independently selected from N, O, S, SO, or SO₂, wherein said ring is optionally fused to a (C6-C10)aryl, (C5-C10)heteroaryl, (C3-C10)cycloalkyl, or a (C3-C10)heterocycloyl;
R⁵ is independently and optionally substituted with one or more R⁷;
R⁶ is selected from: -R⁵, -C(O)R⁵, -C(O)OR⁵, -C(O)N(R⁵)₂ and -S(O)₂R5;
R⁷ is selected from: halogen, -OR⁸, -NO₂, -CN, -CF₃, -OCF₃, -R⁸, oxo, thioxo, 1,2-methylenedioxy, 1,2-ethylenedioxy, -N(R⁸)₂, -SR⁸, -SOR⁸, -SO₂R⁸ -SO₂N(R⁸)₂, -SO₃R⁸, -C(O)R⁸ -C(O)C(O)R⁸, -C(O)CH₂C(O)R⁸, -C(S)R⁸, -C(S)OR⁸, -C(O)OR⁸, -C(O)C(O)OR⁸, -C(O)C(O)N(R⁸)₂, -OC(O)R⁸, -C(O)N(R⁸)₂, -OC(O)N(R⁸)₂, -C(S)N(R⁸)₂, -(CH₂)₀₋₂NHC(O)R⁸, -N(R⁸)N(R⁸)COR⁸, -N(R⁸)N(R⁸)C(O)OR⁸, -N(R⁸)N(R⁸)CON(R⁸)₂, -N(R⁸)SO₂R⁸, -N(R⁸)SO₂N(R⁸)₂, -N(R⁸)C(O)OR⁸, -N(R⁸)C(O)R⁸, -N(R⁸)C(S)R⁸, -N(R⁸)C(O)N(R⁸)₂, -N(R⁸)C(S)N(R⁸)₂, -N(COR⁸)COR⁸, -N(OR⁸)R⁸, -C(=NH)N(R⁸)₂, -C(O)N(OR⁸)R⁸, -C(=NOR⁸)R⁸, -OP(O)(OR⁸)₂, -P(O)(R⁸)₂, -P(O)(OR⁸)₂, or -P(O)(H)(OR⁸); and
R⁸ is selected from: -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂₋C₃H₇, -C(CH₃)₂-CH(CH₃)₂, -C₂H₄-C(CH₃)₃, and -CH(CH₃)-C(CH₃)₃.

11. The compound according to any one of claims 7 - 10, wherein said compound is a uridine precursor, uridine, uridine derivative, UDP or an activator of UDP synthesis, any regulator to increase UDP half-life or an activator of P2Y₆ signaling pathway for use in a therapy for gaining or maintaining weight.

12. The compound according to any one of claims 7 - 11 for use as stimulant of animal weight.

13. A pharmaceutical composition comprising at least one compound of any of the claims 1 - 12 for use in the treatment of a disease related to energy balance and carbohydrate metabolism and homeostasis.

14. A method for screening for a compound for use in the treatment of diseases related to energy balance and carbohydrate metabolism and homeostasis, the method comprising
a) contacting a test compound with P2Y purinoceptor 6 polypeptide,
b) detecting the binding of said test compound to the P2Y purinoceptor 6 polypeptide, and
c) determining the activity of the P2Y purinoceptor 6 polypeptide in the presence of said test compound.

15. The method according to claim 14, wherein instead of polypeptides nucleic acids encoding the polypeptides are used and the expression rate is determined instead of the activity, preferably wherein the test compound is RNA or a peptide or an antibody or a small molecule.
